(19) Europäisches Patentamt
European Patent Office
Office européen des brevets

(11) EP 0 850 940 B1

(12) **FASCICULE DE BREVET EUROPEEN**

(45) Date de publication et mention de la délivrance du brevet:
**16.05.2001 Bulletin 2001/20**

(51) Int Cl.[7]: **C07D 471/04**, A61K 31/435 // (C07D471/04, 221:00, 209:00)

(21) Numéro de dépôt: **97403121.3**

(22) Date de dépôt: **22.12.1997**

(54) **Nouveaux dérivés d'ellipticine, leur procédé de preparation et les compositions pharmaceutiques qui les contiennent**

Ellipticinderivate, ihre Herstellung und diese enthaltende pharmazeutische Zubereitungen

Ellipticine derivatives, their preparation and pharmaceutical compositions containing them

(84) Etats contractants désignés:
**AT BE CH DE DK ES FI FR GB GR IE IT LI LU NL PT SE**

(30) Priorité: **30.12.1996 FR 9616165**

(43) Date de publication de la demande:
**01.07.1998 Bulletin 1998/27**

(73) Titulaire: **ADIR ET COMPAGNIE**
**92415 Courbevoie Cédex (FR)**

(72) Inventeurs:
- **Guillonneau, Claude**
  **92140 Clamart (FR)**
- **Charton, Yves**
  **92330 Sceaux (FR)**
- **Bisagni, Emile**
  **91400 Orsay (FR)**
- **Atassi, Ghanem**
  **92210 Saint Cloud (FR)**
- **Pierre, Alain**
  **78580 Les Alluets Le Roi (FR)**
- **Guilbaud, Nicolas**
  **78170 La Celle Saint Cloud (FR)**

(56) Documents cités:
**EP-A- 0 591 058**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 850 940 B1

Printed by Jouve, 75001 PARIS (FR)

## Description

La présente invention concerne de nouveaux composés d'olivacine ou d'éllipticine, leur procédé de préparation et les compositions pharmaceutiques qui les contiennent.

Les composés de la présente invention trouvent leur utilisation en thérapeutique grâce à leur activité antitumorale.

Certains dérivés de la famille de l'olivacine ou de l'ellipticine sont déjà connus pour leurs propriétés anticancéreuses, cf la demande de brevet EP 0 591 058 A1.

Les besoins de la thérapeutique exigent le développement constant de nouveaux agents anticancéreux dans le but d'obtenir des molécules à la fois plus actives et mieux tolérées.

La présente invention concerne des dérivés de l'olivacine ou de l'ellipticine, présentant, par rapport aux composés les plus proches de l'art antérieur, une originalité structurale (puisque la substitution sur la position 9 du tétracycle diffère de celle des dérivés connus), une originalité toxicologique (les dérivés de la présente invention présentant une meilleure tolérance que celle des composés décrits dans l'art antérieur), ainsi qu'une meilleure activité "in vivo" par rapport aux produits de référence.

La présente invention a plus particulièrement pour objet :

. les dérivés de formule I:

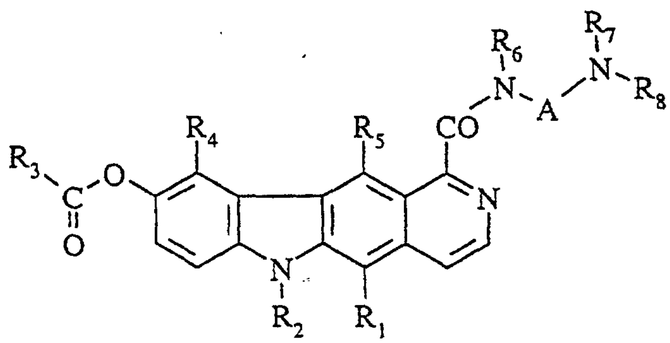

(I)

dans laquelle :

**$R_1$** représente un radical alkyle de 1 à 6 atomes de carbone en chaine droite ou ramifiée ;

**$R_2$, $R_5$ et $R_6$,** identiques ou différents représentent chacun un atome d'hydrogène ou un radical alkyle, de 1 à 6 atomes de carbone, en chaine droite ou ramifiée;

**$R_3$** représente :

A) un atome d'hydrogène,

B) un groupe $(C_3\text{-}C_8)$cycloalkyle, un groupe $(C_1\text{-}C_{20})$alkyle, $(C_2\text{-}C_{20})$-alkényle ou $(C_4\text{-}C_{20})$-alkadiényle, chacun d'eux en chaine droite ou ramifiée, et chacun de ces 4 groupes étant non substitué ou substitué par 1 à 3 groupes choisis parmi les radicaux :

a) $(C_1\text{-}C_6)$alkoxy ;

b) carboxy, $(C_1\text{-}C_6)$alkoxy-carbonyle, et benzyloxycarbonyle ;

c) aryle mono- bi- ou tri-cyclique, ou hétéroaryle renfermant de 1 à 3 hétéroatomes choisis parmi les atomes d'azote, oxygène et soufre, chacun d'eux partiellement ou totalement hydrogéné ;

C) un groupe aryle mono- bi- ou tri- cyclique éventuellement totalement ou partiellement hydrogéné, ou un groupe hétéroaryle, composé de 1 à 3 cycles penta et hexagonaux et comportant de 1 à 3 hétéroatomes choisis parmi les atomes d'azote, oxygène et soufre, tel que par exemple un groupe pyridyle, pipéridyle, pipérazinyle, pyrimidinyle, thiényle, benzothiényle, furyle, pyranyle, benzofuranyle, chroményle, chromanyle, pyrrolyle, imidazolyle ou quinolyle ;

chacun des groupes aryle et hétéroaryle ci-dessus définis pouvant être éventuellement

substitué par 1 à 3 substituants choisis parmi les radicaux :

a) $(C_1-C_6)$alkoxycarbonyle,
b) benzyloxycarbonyle,
c) $(C_1-C_6)$alkyle en chaine linéaire et ramifiée, non substitué et substitué par un et deux groupes phényles,
d) $(C_1-C_6)$alkoxy en chaine linéaire et ramifiée, et
e) carboxy ;

D) un groupe amino- mono- ou di-substitué par un ou deux substituants choisis parmi les radicaux :

a) $(C_1-C_6)$alkyles non substitués et substitués par un et deux substituants choisis chacun parmi les radicaux : carboxy, $(C_1-C_6)$ alkoxycarbonyle et benzyloxycarbonyle,
b) aryle et hétéroaryle chacun mono- et bicyclique, ainsi que chacun d'eux partiellement et totalement hydrogénés tous étant non substitués et substitués par un et deux groupes choisis chacun parmi les radicaux carboxy, $(C_1-C_6)$alkoxycarbonyle et benzyloxycarbonyle ;

E) un groupe amino inclus dans un cycle penta ou hexagonal, renfermant éventuellement un second hétéroatome choisi parmi les atomes d'azote, oxygène et soufre, et éventuellement substitué par un ou deux groupes choisis chacun parmi les radicaux $(C_1-C_6)$alkyle, benzhydryle, $(C_1-C_6)$alkoxy, $(C_1-C_6)$alkoxycarbonyle, benzyloxycarbonyle, carboxy et pipéridino ;

**$R_4$** représente :

- un atome d'hydrogène,
- un radical $(C_1-C_6)$ alkyle, en chaine droite ou ramifiée éventuellement substitué par un groupe di-$(C_1-C_6)$alkylamino, ou
- un radical $(C_2-C_6)$-alkène;

**$R_7$ et $R_8$**, identiques ou différents représentent chacun un atome d'hydrogène, un radical $(C_1-C_6)$alkyle ou $(C_2-C_6)$-alkényle, chacun en chaine droite ou ramifiée, et
$R_7$ et $R_8$ peuvent ensemble avec l'atome d'azote auquel ils sont liés former un hétérocycle, renfermant éventuellement un second hétéroatome choisi parmi les atomes d'azote, oxygène et soufre, tel que par exemple les noyaux pyrrole, pyroline, pyrolidine, imidazole, imidazoline, imidazolidine, oxazole, oxazoline, oxazolidine, pyridine, pipéridine, morpholine, thiomorpholine, pipérazine, pyrazole, pyrazoline, pyridazine, pyrimidine et pyrazine, et de plus $R_7$ peut être lié à $R_6$ afin de former ensemble un pont $-(CH_2)_m-$dans lequel m prend les valeurs 2 ou 3 ; et

**A** représente une chaine hydrocarbonée saturée linéaire ou ramifiée contenant de 1 à 10 atomes de carbone,

ainsi que leurs éventuels isomères optiques, N-oxydes et sels d'addition, à un acide ou à une base, pharmaceutiquement acceptables.

L'invention a également pour objet le procédé de préparation des composés de formule I caractérisé en ce que :

- ♦ on estérifie un composé de formule II:

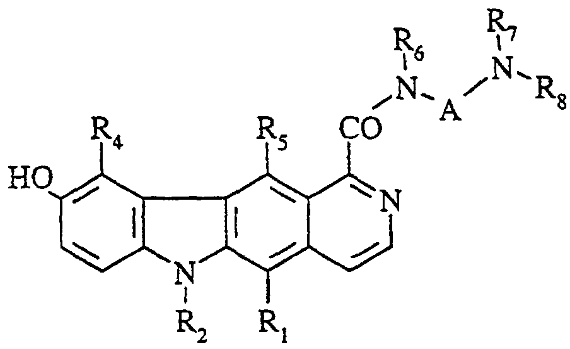

(II)

dans laquelle :

$R_1$, $R_2$, $R_4$, $R_5$, $R_6$, $R_7$, $R_8$ et A ont les significations précédemment définies au moyen d'un composé choisi parmi ceux correspondant aux formules :

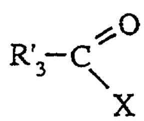

(IIIa)

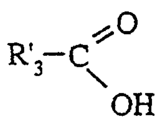

(IIIb)

et

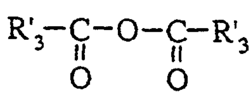

(IIIc)

dans lesquelles :

X représente un atome ou un groupe labile tel que par exemple un atome d'halogène, et

$R'_3$ représente :

α) un groupe $(C_3\text{-}C_8)$cycloalkyle, un groupe $(C_1\text{-}C_{20})$alkyle, $(C_2\text{-}C_{20})$-alkényle ou $(C_4\text{-}C_{20})$ -alkadiényle, chacun d'eux en chaine droite ou ramifiée, et chacun de ces 4 groupes étant non substitué ou substitué par 1 à 3 groupes choisis parmi les radicaux :

a) $(C_1\text{-}C_6)$alkoxy ;

b) $(C_1\text{-}C_6)$alkoxycarbonyle, et benzyloxycarbonyle ; et

c) aryle mono- bi- ou tri-cyclique, ou hétéroaryle renfermant de 1 à 3 hétéroatomes choisis parmi les atomes d'azote, oxygène et soufre ;

β) un groupe aryle mono- bi- ou tri- cyclique éventuellement totalement ou partiellement hydrogéné, ou un groupe hétéroaryle, composé de 1 à 3 cycles penta et hexagonaux et comportant de 1 à 3 hétéroatomes choisis parmi les atomes d'azote, oxygène et soufre, tel que par exemple un groupe pyridyle,

pipéridyle, pipérazinyle, pyrimidinyle, thiényle, benzothiényle, furyle, pyranyle, benzofuranyle, chroményle, chromanyle, pyrrolyle, imidazolyle ou quinolyle ;
chacun des groupes aryle et hétéroaryle ci-dessus définis pouvant être éventuellement substitué par 1 à 3 substituants choisis parmi les radicaux :

a) ($C_1$-$C_6$)alkoxycarbonyle,
b) benzyloxycarbonyle,
c) ($C_1$-$C_6$)alkyle en chaine linéaire et ramifiée, non substitué et substitué par un et deux groupes phényles, et
d) ($C_1$-$C_6$)alkoxy en chaîne linéaire et ramifiée ;

γ) un groupe amino- di-substitué par deux substituants choisis chacun parmi les radicaux :

a) ($C_1$-$C_6$)alkyles non substitués et substitués par un et deux substituants choisis chacun parmi les radicaux : ($C_1$-$C_6$) alkoxycarbonyle et benzyloxycarbonyle, et
b) aryle et hétéroaryle chacun mono- et bicyclique, ainsi que chacun d'eux partiellement et totalement hydrogénés tous étant non substitués et substitués par un et deux groupes choisis chacun parmi les radicaux ($C_1$-$C_6$) alkoxycarbonyle et benzyloxycarbonyle ; et

δ) un groupe amino inclus dans un cycle penta ou hexagonal, renfermant éventuellement un second hétéroatome choisi parmi les atomes d'azote, oxygène et soufre, et éventuellement substitué par un ou deux groupes choisis chacun parmi les radicaux ($C_1$-$C_6$)alkyle, benzhydryle, ($C_1$-$C_6$)alkoxy, ($C_1$-$C_6$) alkoxycarbonyle, benzyloxycarbonyle et pipéridino ;

pour obtenir les composés de formule Ia :

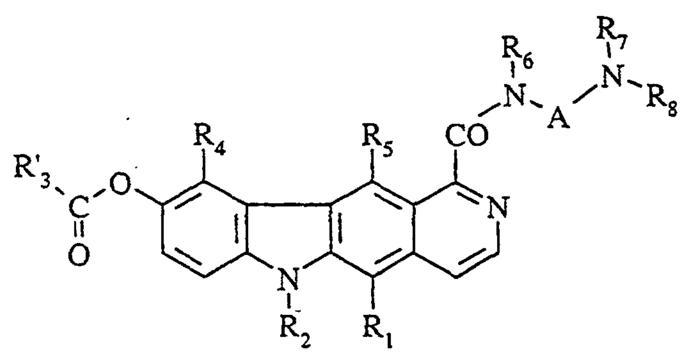

(Ia)

dans laquelle $R_1$, $R_2$, $R'_3$, $R_4$, $R_5$, $R_6$, $R_7$, $R_8$ et A ont les significations précédemment définies :

- on traite :
  les composés de formule Ia dans laquelle $R'_3$ renferme comme substituant au moins un groupe benzyloxy, c'est à dire les composés répondant plus précisément à la formule I'a :

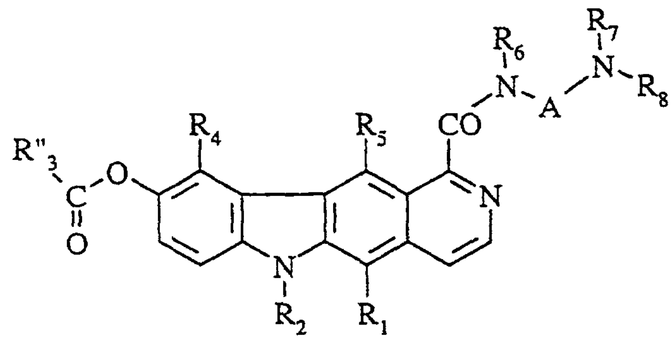

(I'a)

dans laquelle :

$R_1$, $R_2$, $R_4$, $R_5$, $R_6$, $R_7$, $R_8$ et A ont les significations précédemment définies et $R''_3$ représente :

a) un groupe $(C_3-C_8)$cycloalkyle, un groupe $(C_1-C_{20})$alkyle en chaîne droite ou ramifiée, chacun de ces groupes étant substitué par 1 à 3 radicaux benzyloxycarbonyles ;
β) un groupe aryle mono- bi- ou tri- cyclique éventuellement totalement ou partiellement hydrogéné, ou un groupe hétéroaryle, composé de 1 à 3 cycles penta et hexagonaux et comportant de 1 à 3 hétéroatomes choisis parmi les atomes d'azote, oxygène et soufre, tel que par exemple un groupe pyridyle, pipéridyle, pipérazinyle, pyrimidinyle, thiényle, benzothiényle, furyle, pyranyle, benzofuranyle, chroményle, chromanyle, pyrrolyle, imidazolyle ou quinolyle ;
chacun des groupes aryle et hétéroaryle ci-dessus définis étant substitué par 1 à 3 radicaux benzyloxycarbonyles ;
γ) un groupe amino- mono- ou di-substitué par un ou deux substituants choisis parmi les radicaux :

a) $(C_1-C_6)$alkyles substitués par un et deux radicaux benzyloxycarbonyles,
b) aryle et hétéroaryle chacun mono- et bicyclique, ainsi que chacun d'eux partiellement et totalement hydrogénés tous étant substitués par un et deux radicaux benzyloxycarbonyles ;

δ) un groupe amino inclus dans un cycle penta ou hexagonal, renfermant éventuellement un second hétéroatome choisi parmi les atomes d'azote, oxygène et soufre, et substitué par un ou deux radicaux benzyloxycarbonyles,

avec de l'hydrogène ou un donneur d'hydrogène (tel que, par exemple, le cyclohexène) pour obtenir les composés correspondants dans lesquels tout substituant benzyloxycarbonyle dans le composé de départ de formule I'a est remplacé par un radical carboxy, c'est à dire pour obtenir les composés de formule Ib :

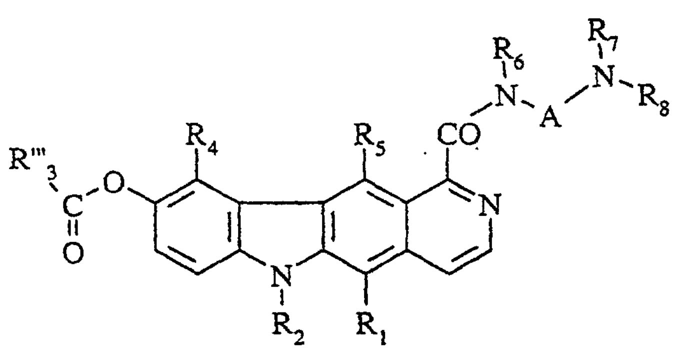

(Ib)

dans laquelle :

$R_1$, $R_2$, $R_4$, $R_5$, $R_6$, $R_7$, $R_8$ et A ont les significations précédemment définies et $R'''_3$ représente :

α) un groupe $(C_3-C_8)$cycloalkyle, un groupe $(C_1-C_{20})$alkyle en chaine droite ou ramifiée, chacun de ces groupes étant substitué par 1 à 3 radicaux carboxy,
β) un groupe aryle mono- bi- ou tri- cyclique éventuellement totalement ou partiellement hydrogéné, ou un groupe hétéroaryle, composé de 1 à 3 cycles penta et hexagonaux et comportant de 1 à 3 hétéroatomes choisis parmi les atomes d'azote, oxygène et soufre, tel que par exemple un groupe pyridyle, pipéridyle, pipérazinyle, pyrimidinyle, thiényle, benzothiényle, furyle, pyranyle, benzofuranyle, chroményle, chromanyle, pyrrolyle, imidazolyle ou quinolyle ;
chacun des groupes aryle et hétéroaryle ci-dessus définis étant substitué par 1 à 3 radicaux carboxy ;
γ) un groupe amino- mono- ou di-substitué par un ou deux substituants choisis parmi les radicaux :

a) $(C_1-C_6)$alkyles substitués par un et deux radicaux carboxy,
b) aryle et hétéroaryle chacun mono- et bicyclique, ainsi que chacun d'eux partiellement et totalement hydrogénés tous étant substitués par un et deux radicaux carboxy ;

δ) un groupe amino inclus dans un cycle penta ou hexagonal, renfermant éventuellement un second hétéroatome choisi parmi les atomes d'azote, oxygène et soufre, et substitué par un ou deux radicaux carboxy ; et

- ♦ on fait réagir un composé de formule II telle que précédemment définie, avec un mélange d'acide formique et d'anhydride acétique pour obtenir les composés de formule I dans laquelle $R_3$ représente un atome d'hydrogène c'est à dire pour obtenir les composés de formule Ic:

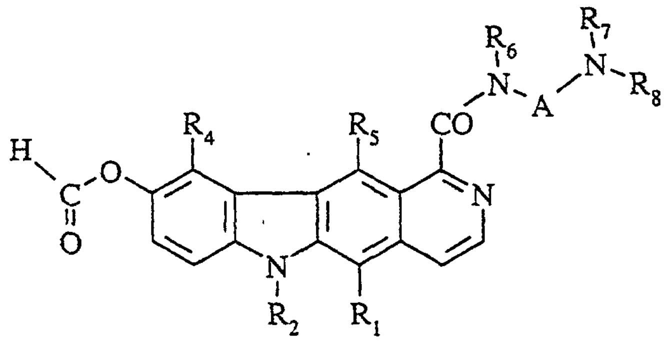

(Ic)

dans laquelle $R_1$, $R_2$, $R_4$, $R_5$, $R_6$, $R_7$, $R_8$ et A ont les significations précédemment définies.

L'ensemble des composés de formule Ia, Ib et Ic forme l'ensemble des composés de formule I.
La présente invention a également pour objet le procédé de préparation :

- des composés de formule I dans laquelle la signification de $R_3$ se limite à un radical $(C_1\text{-}C_{20})$alkyle, substitué par un radical carboxy, c'est à dire :

  - des composés répondant plus spécifiquement à la formule I' (elle-même incluse dans la formule Ib) :

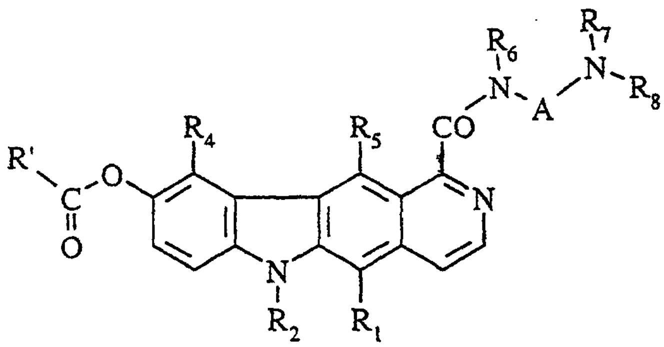

(I')

dans laquelle :

. $R_1$, $R_2$, $R_4$, $R_5$, $R_6$, $R_7$, $R_8$ et A ont les significations précédemment définies et
. R' représente un radical $(C_1\text{-}C_{20})$alkyle, substitué par un radical carboxy,

caractérisé en ce que l'on traite

- les composés de formule I":

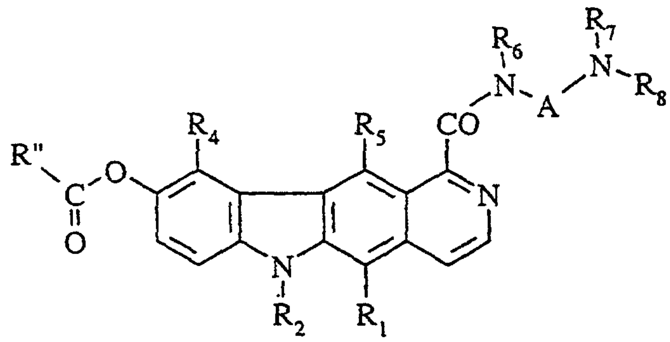

(I")

dans laquelle :

- . $R_1$, $R_2$, $R_4$, $R_5$, $R_6$, $R_7$, $R_8$ et A ont les significations précédemment définies et
- . R" représente un radical ($C_1$-$C_{20}$)alkyle, substitué en bout de chaîne par un radical benzyloxy,

- avec de l'hydrogène ou un donneur d'hydrogène (tel que par exemple le cyclohexène).

Certains composés de formule I' peuvent être obtenus en faisant réagir un composé de formule II avec un anhydride cyclique de formule :

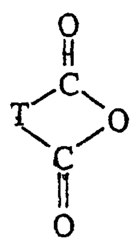

dans laquelle T représente une chaine hydrocarbonée saturée de 2 à 10 atomes de carbone, éventuellement substituée par un ou deux radicaux ($C_1$-$C_5$)alkyle.

Ce procédé fait également partie de la présente invention.

La présente invention a aussi pour objet le procédé de préparation :

- des composés de formule I dans laquelle la signification de $R_3$ se limite à un groupe amino monosubstitué par : soit un radical ($C_1$-$C_6$)alkyle non substitué ou substitué par un ou deux substituants choisis parmi les radicaux ($C_1$-$C_6$)alkoxycarbonyle et benzyloxycarbonyle,

soit un radical aryle ou hétéroaryle mono ou bi-cyclique éventuellement totalement ou partiellement hydrogéné, non substitué ou substitué par un ou deux groupes choisis chacun parmi les radicaux ($C_1$-$C_6$)alkoxycarbonyle et benzyloxycarbonyle, c'est à dire des composés répondant plus spécifiquement à la formule I'" (elle-même incluse dans la formule Ia) :

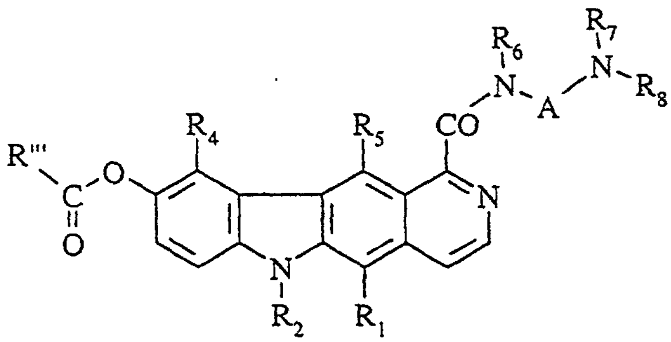

(I''')

dans laquelle :

- $R_1$, $R_2$, $R_4$, $R_5$, $R_6$, $R_7$, $R_8$ et A ont les significations précédemment définies et
- R''' représente un groupe amino monosubstitué par :

    soit un radical ($C_1$-$C_6$)alkyle non substitué ou substitué par un ou deux substituants choisis parmi les radicaux ($C_1$-$C_6$)alkoxycarbonyle et benzyloxycarbonyle,
    soit un radical aryle ou hétéroaryle mono- ou bi-cyclique éventuellement totalement ou partiellement hydrogéné non substitué ou substitué par un ou deux groupes choisis chacun parmi les radicaux ($C_1$-$C_6$)alkoxycarbonyle et benzyloxycarbonyle,

caractérisé en ce que l'on fait réagir :

- un composé de formule II précédemment définie,
- avec un isocyanate de formule IV :

$$R_9\text{-N=C=O} \qquad \text{(IV)}$$

dans laquelle :

- $R_9$ représente :

    soit un radical ($C_1$-$C_6$)alkyle non substitué ou substitué par un ou deux substituants choisis parmi les radicaux ($C_1$-$C_6$)alkoxycarbonyle et benzyloxycarbonyle,
    soit un radical aryle ou hétéroaryle mono- ou bi-cyclique éventuellement totalement ou partiellement hydrogéné, non substitué ou substitué par un ou deux groupes choisis chacun parmi les radicaux ($C_1$-$C_6$) alkoxycarbonyle et benzyloxycarbonyle.

Il est particulièrement avantageux de faire réagir les composés de formule IIIa, flic et IV avec le composé de formule II, en présence éventuelle d'un accepteur de l'hydracide formé au cours de la réaction, dans un solvant tel que, par exemple, le tétrahydrofurane, le dioxane, le dichlorométhane, le dichlorobenzène, le diméthylformamide ou la pyridine, à une température entre 20 et 80 °C. Comme accepteur, on peut utiliser, par exemple, un carbonate alcalin tel que le carbonate de potassium, une amine tertiaire telle que la triéthylamine ou la diméthylaminopyridine (D.M.A.P) ou le 1,8-diazabicyclo[5,4,0]undec-7-ène (D.B.U.).

La réaction des composés de formule II avec les composés de formule IIIb s'effectue de manière adéquate, en présence d'un agent de couplage tel que le dicyclohexylcarbodiimide éventuellement en présence de 1-hydroxybenzotriazole, le 1-éthyl-3-(3-diméthylaminopropyl) carbodiimide ou le 1,1-carbonyl diimidazole, dans un solvant aprotique comme le tétrahydrofurane ou le diméthylformamide.

La réaction des composés de formule I'a avec l'hydrogène ou un donneur d'hydrogène tel que le cyclohexène, s'effectue avantageusement dans un solvant tel que l'eau, l'éthanol ou le diméthylformamide, en présence d'un catalyseur d'hydrogénation tel que le palladium sur charbon ou d'hydroxyde de palladium, sous une pression comprise entre $1.10^5$Pa et $5.10^5$ Pa, à une température comprise entre 20 et 80 °C.

Les composés de formule II utilisés comme matières premières sont soit décrits dans le brevet EP 0 591 058

$A_1$, soit préparés à partir de produits connus comme décrit dans le brevet EP 0 591 058 $A_1$ pour préparer des produits analogues.

Les autres composés utilisés comme matières premières sont soit des produits commerciaux soit des produits préparés à partir de substances connues, selon des méthodes de synthèse organique classiques déjà décrites dans la littérature.

Les dérivés de formule I donnent des sels avec des acides ou des bases physiologiquement tolérables, sels qui sont à ce titre inclus dans la présente invention.

Les dérivés de formule I donnent aussi des composés N-oxydes qui, à ce titre, sont également inclus dans la présente invention.

Certains dérivés de formule I renferment un ou plusieurs carbone asymétrique et de ce fait donnent des énantiomères ou des diastéréoisomères qui font également partie de la présente invention.

Les dérivés de la présente invention présentent des propriétés pharmacologiques particulièrement intéressantes, notamment une excellente cytotoxicité in vitro et une activité antitumorale in vivo supérieure à celle des produits de l'état antérieur de la technique, ce qui, ajouté au fait que ces dérivés sont particulièrement bien tolérés permet leur utilisation en thérapeutique en tant qu'agents antitumoraux.

Sont particulièrement intéressants et représentatifs de l'invention les composés de formule I dans laquelle :

- $R_1$ et $R_2$ représentent chacun un radical méthyle ;
- $R_3$ représente un groupe carboxyalkyle dans lequel la partie alkyle renferme de 1 à 8 atomes de carbone en chaîne droite ou ramifiée ;
- $R_4$ et $R_5$, identiques ou différents, représentent chacun un atome d'hydrogène ou un radical méthyle ;
- $R_6$ représente un atome d'hydrogène ;
- $R_7$ et $R_8$ représentent chacun un radical méthyle et
- A représente $-(CH_2)_2-$ ou $(CH_2)_3$;

leurs éventuels isomères optiques, N-oxydes et sels d'addition, à un acide ou une base, pharmaceutiquement acceptables.

La présente invention a également pour objet les compositions pharmaceutiques contenant comme principe actif au moins un composé de la présente invention mélangé ou associé à un ou plusieurs excipients pharmaceutiques ou véhicules inertes non toxiques.

Ces compositions pharmaceutiques sont généralement présentées sous forme dosée convenant pour l'administration par voie orale, rectale ou parentérale et notamment les comprimés, dragées, gélules, suppositoires et solutions injectables ou buvables.

La posologie varie selon l'âge et le poids du patient, la voie d'administration, la nature de l'indication thérapeutique et les traitements éventuellement associés et s'échelonne entre 0,1 et 400 mg par jour en une ou plusieurs administrations.

Les exemples suivants illustrent la présente invention, les points de fusion étant déterminés à la platine de Kofler (K) ou au tube capillaire (cap).

La silice utilisée pour les purifications est la silice Amicon (0,035-0,07 mm). La pression utilisée est de $10^5$ Pa.

__Exemple 1__

Le 1-[(2-diméthylaminoéthyl)aminocarbonyl]-5,6-diméthyl-9-tétradécanoyloxy-6H-pyrido [4,3-b] carbazole

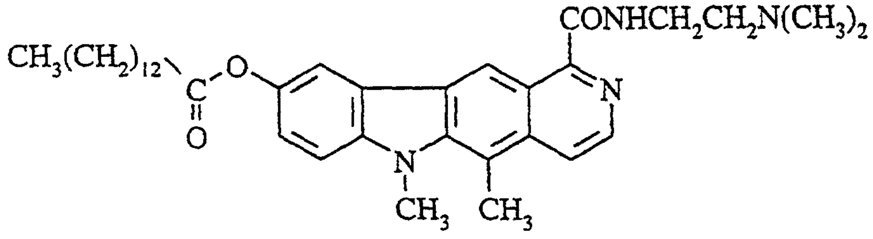

On dissous 2g de 1-[(2-diméthylaminoéthyl)aminocarbonyl]-5,6-diméthyl-9-hydroxy-6H-pyrido[4,3-b]carbazole dans 60 ml de pyridine. On refroidit à 5 °C puis on coule 4,67 g d'anhydride myristique en 3 minutes. On agite 2 heures à 5 °C puis 16 heures à température ambiante. On concentre sec à température inférieure à 40 °C. On chromatographie le résidu sur 150 g de silice en éluant avec un mélange de dichlorométhane et de méthanol. On obtient 2,4 g de produit souhaité, PF(K) : 98 °C.

**Exemples 2 à 10**

En opérant comme décrit dans l'exemple 1, ont été préparé les composés objet des exemples suivants :

2) Le 1-[(2-diméthylaminoéthyl)aminocarbonyl]-5,6-diméthyl-9-acétoxy-6H-pyrido[4,3-b] carbazole, PF(K) : 166 °C.

3) Le dichlorhydrate de 1-[(2-diméthylaminoéthyl)aminocarbonyl]-5,6-diméthyl-9-(4-carboxybutanoyloxy)-6H-pyrido[4,3-b]carbazole, PF(cap) : 190-200 °C.

4) Le dichlorhydrate de 1-[(2-diméthylaminoéthyl)aminocarbonyl]-5,6-diméthyl-9-(5-carboxypentanoyloxy)-11-méthyl-6H-pyrido[4,3-b]carbazole, PF(cap) : 180 °C (décomposition).

5) Le dichlorhydrate de 1-[(2-diméthylaminoéthyl)aminocarbonyl]-5,6-diméthyl-9-(6-carboxyhexanoyloxy)-6H-pyrido[4,3-b]carbazole, PF : 190-196 °C.

6) Le dichlorhydrate de 1-[(2-diméthylaminoéthyl)aminocarbonyl]-5,6-diméthyl-9-(7-carboxy heptanoyloxy)-6H-pyrido[4,3-b]carbazole, PF(cap) : 210-215 °C.

7) Le dichlorhydrate de 1-[(2-diméthylaminoéthyl)aminocarbonyl]-5,6-diméthyl-9-(4-carboxy-3,3-diméthyl butanoyloxy)-6H-pyrido[4,3-b]carbazole, PF(cap) : 190 °C (décomposition).

8) Le dichlorhydrate de 1-[(2-diméthylaminoéthyl)aminocarbonyl]-5,6,10-triméthyl-9-(5-carboxypentanoyloxy)-6H-pyrido[4,3-b]carbazole, PF(cap) : 215-225 °C.

9) Le dichlorhydrate de 1-[(2-diméthylaminoéthyl)aminocarbonyl]-5,6,11-triméthyl-9-(5-carboxypentanoyloxy)-6H-pyrido[4,3-b]carbazole, PF(cap) : 185-195 °C.

10) Le 1-[(2-diméthylaminoéthyl)aminocarbonyl]-5,6-diméthyl-9-(4-carboxy-3-méthyl butanoyloxy)-6H-pyrido-[4,3-b]carbazole, PF (cap) : 170-175 °C.

**Exemple 11**

Le (R,S)-1-[(2-diméthylaminoéthyl)aminocarbonyl]-5,6-diméthyl-9-(trans phénylcyclopropyl carboxy)-6H-pyrido[4,3-b] carbazole

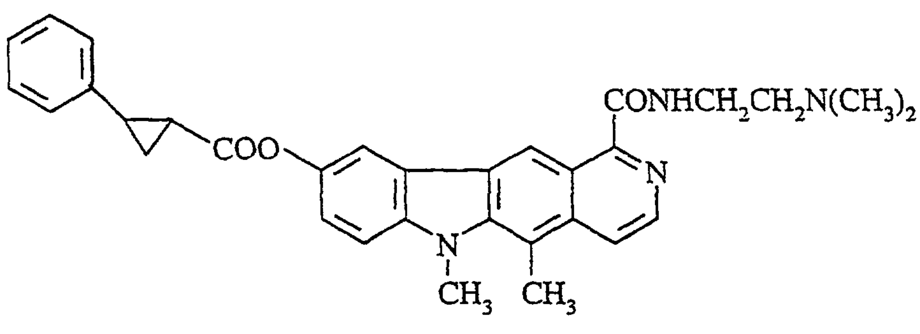

On dissous 1 g de 1-[(2-diméthylaminoéthyl)aminocarbonyl]-5,6-diméthyl-9-hydroxy-6H-pyrido[4,3-b]carbazole et 0,29 g de triéthylamine dans 50 ml de tétrahydrofurane. On refroidit à 5 °C et on coule en 15 minutes 0,53 g

de chlorure de l'acide (R,S) trans 2-phénylcyclopropylcarboxylique dissous dans 5 ml de tétrahydrofurane. On agite 1 heure à 5 °C puis 20 heures à température ambiante. On concentre à sec. On reprend le résidu dans le dichlorométhane, lave à l'eau, sèche sur sulfate de sodium. On concentre à sec. On chromatographie sur 70 g de silice en éluant avec un mélange dichlorométhane et de méthanol (90-10). On obtient 0,9 g de produit souhaité, PF(cap) : 176-178 °C.

**Exemples 12 à 15**

En opérant comme décrit dans l'exemple 11, ont été préparé les composés objet des exemples suivants :

12) Le 1-[(2-diméthylaminoéthyl)aminocarbonyl]-5,6-diméthyl-9-(4-méthoxycarbonyl butanoyl oxy)-6H-pyrido [4,3-b]carbazole, PF(cap) : 120-122 °C.

13) Le 1-[(2-diméthylaminoéthyl)aminocarbonyl]-5,6-diméthyl-9-(2-phénylacétoxy)-6H-pyrido[4,3-b]carbazole, PF (cap) : 84-86 °C.

14) Le 1-[(2-diméthylaminoéthyl)aminocarbonyl]-5,6-diméthyl-9-nonanoyloxy-6H-pyrido[4,3-b]carbazole, PF (cap) : 122-125 °C.

15) Le 1-[(2-diméthylaminoéthyl)aminocarbonyl]-5,6-diméthyl-9-[3-éthoxypropanoyloxy]-6H-pyrido[4,3-b]carbazole, PF(cap) : 116-118 °C.

**Exemple 16**

Le 1-[(2-diméthylaminoéthyl)aminocarbonyl]-5,6-diméthyl-9-[3-benzyloxycarbonylpropanoyloxy]-6H-pyrido[4,3-b]carbazole

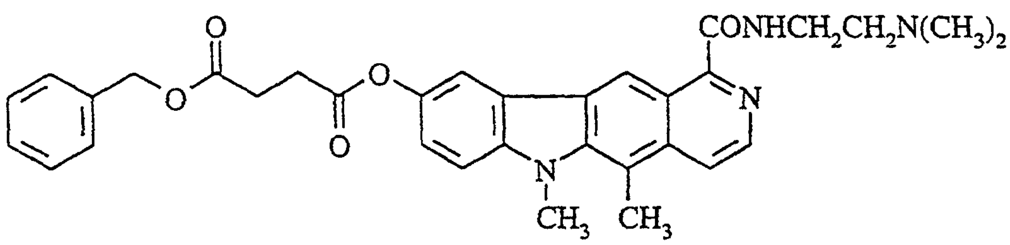

On agite 4,5 g de monoester benzylique de l'acide succinique, 4,95 g de dicyclohexylcarbodiimide, 2,85 g de 1-hydroxybenzotriazole et 240 ml de diméthylformamide pendant 4 heures à température ambiante. On ajoute 3 g de 1-[(2-diméthylaminoéthyl)aminocarbonyl]-5,6-diméthyl-9-hydroxy-6H-pyrido[4,3-b]carbazole et on agite 16 heures à température ambiante. On concentre à sec et reprend le résidu dans le dichlorométhane. On lave avec une solution saturée de bicarbonate de sodium, sèche sur sulfate de sodium et concentre à sec. On chromatographie le résidu sur 280 g de silice en éluant avec un mélange de toluène, d'éthanol et de triéthylamine (90-10-0,5). On concentre à sec les fractions intéressantes et on reprend le résidu dans l'éthanol. On concentre à nouveau à sec. On reprend le résidu dans l'éther, essore et sèche à 60 °C sous 13,328 Pa. On obtient 3,3 g de produit souhaité, PF(cap) : 174-176 °C.

**Exemples 17 et 18**

En opérant comme décrit dans l'exemple 16, ont été préparés les composés objet des exemples suivants :

17) Le 1-[(2-diméthylaminoéthyl)aminocarbonyl]-5,6-diméthyl-9-(5-benzyloxycarbonyl pentanoyloxy)-6H-pyrido [4,3-b]carbazole, PF(cap) : 88-90 °C.

18) Le (R,S) {[1-(2-diméthylaminoéthyl)aminocarbonyl]-5,6-diméthyl-6H-pyrido[4,3-b] carbazol-9-yl}ester de l'acide 5-[di[1,2]thiolan-3-yl] pentanoïque, PF(cap) : 140-142 °C.

**Exemple 19**

Dichlorhydrate du 1-[(N-oxyde du 2-diméthylaminoéthyl)aminocarbonyl]-5,6-diméthyl-9-acétoxy-6H-pyrido[4,3-b]carbazole

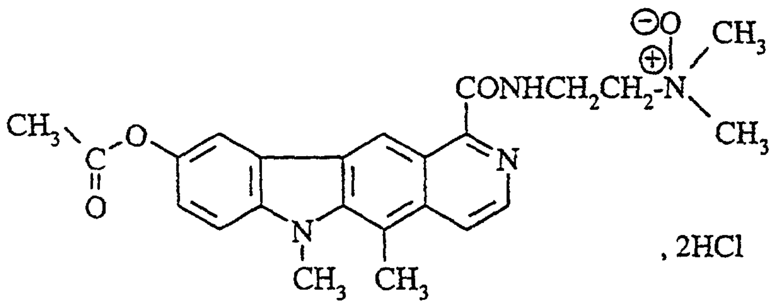

La réaction du 1-[(2-diméthylaminoéthyl)aminocarbonyl]-5,6-diméthyl-9-acétoxy-6H-pyrido[4,3-b]carbazole avec l'acide 3-chloroperoxybenzoïque dans le dichlorométhane à température ambiante conduit au produit souhaité, PF(cap) : 120 °C (décomposition).

**Exemple 20**

1-[(2-diméthylaminoéthyl)aminocarbonyl]-5,6-diméthyl-9-(4-pipéridino pipéridino carbonyl oxy)-6H-pyrido[4,3-b]carbazole

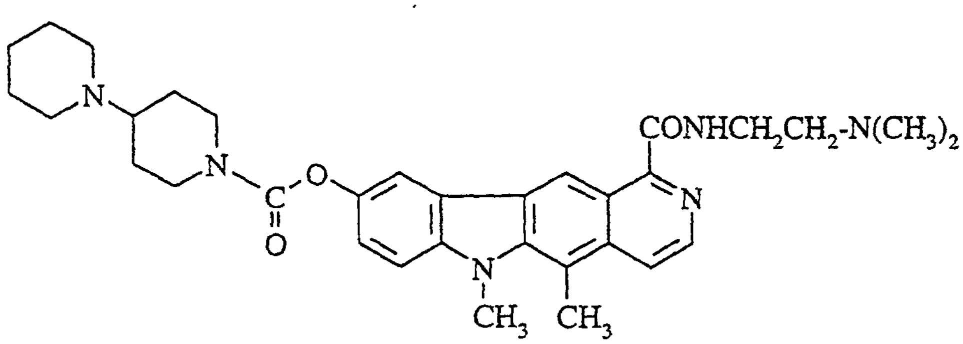

On agite 1,6 g de 1-[(2-diméthylaminoéthyl)aminocarbonyl]-5,6-diméthyl-9-hydroxy-6H-pyrido[4,3-b]carbazole et 1,5 g de N-chloroformiate de 4-pipéridino pipéridine dans 80 ml de pyridine pendant 16 heures. On concentre à sec, reprend dans le dichlorométhane, lave avec une solution à 10 % de bicarbonate de sodium, sèche sur sulfate de sodium et concentre à sec.

On ajoute du dioxane et concentre à nouveau afin d'éliminer la pyridine résiduelle. On triture dans un mélange d'acétate d'éthyle et d'éther. On essore, lave à l'éther et sèche à 50 °C sous 66 Pa. On obtient 2,3 g de produit souhaité, PF(cap) : 190-200 °C.

**Exemples 21 à 27**

En opérant comme décrit dans l'exemple 20, ont été préparés les composés objet des exemples suivants :

21) Le 1-[(2-diméthylaminoéthyl)aminocarbonyl]-5,6-diméthyl-9-(N-méthyldodécylamino carbonyloxy)-6H-pyrido [4,3-b]carbazole, PF(cap) : 90-93 °C.

22) Le 1-[(2-diméthylaminoéthyl)aminocarbonyl]-5,6-diméthyl-9-(N-phényl-N-méthylamino carbonyloxy)-6H-pyrido[4,3-b]carbazole, PF(cap) : 125-128 °C.

23) Le 1-[(2-diméthylaminoéthyl)aminocarbonyl]-5,6-diméthyl-9-diméthylamino carbonyloxy- 6H-pyrido[4,3-b]carbazole, PF(cap) : 188-190 °C.

24) Le 1-[(2-diméthylaminoéthyl)aminocarbonyl]-5,6-diméthyl-9-morpholinocarbonyloxy-6H-pyrido[4,3-b]carbazole, PF(cap) : 190-192 °C.

25) Le 1-[(2-diméthylaminoéthyl)aminocarbonyl]-5,6-diméthyl-9-[(4-benzhydrylpérazin-1-yl)carbonyloxy]-6H-pyrido[4,3-b]carbazole, PF(cap) : 135-138 °C.

26) Le 1-[(2-diméthylaminoéthyl)aminocarbonyl]-5,6-diméthyl-9-(4-méthylpipérazino carbonyloxy)-6H-pyrido [4,3-b]carbazole, PF(cap) : 134-137 °C.

27) Le 1-[(2-diméthylaminoéthyl)aminocarbonyl]-5,6-diméthyl-9-(4-benzyloxycarbonyl pipérazinocarbonyloxy)-6H-pyrido[4,3-b]carbazole, PF(cap) : 186-188 °C.

**Exemple 28**

1-[(2-diméthylaminoéthyl)aminocarbonyl]-5,6-diméthyl-9-(docécylaminocarbonyloxy)-6H-pyrido[4,3-b]carbazole

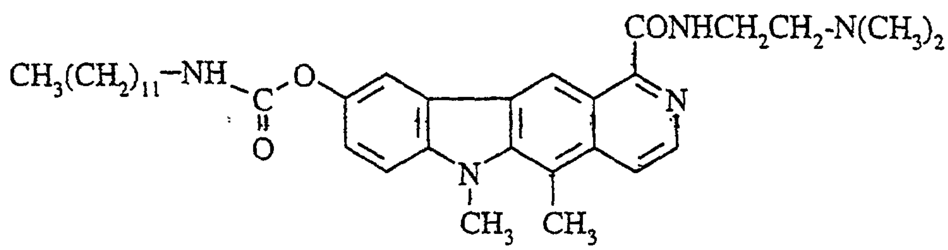

On agite 3,8 g de 1-[(2-diméthylaminoéthyl)aminocarbonyl]-5,6-diméthyl-9-hydroxy-6H-pyrido[4,3-b]carbazole, 3,2 g d'isocyanate de n dodécyle, 0,05 g de 1,8-diazabicyclo[5,4,0]undec-7-ène dans 50 ml de pyridine pendant 16 heures à 50 °C. On concentre à sec, ajoute du dioxane et concentre à nouveau. On chromatographie sur 480 g de silice en éluant avec dichlorométhane, méthanol et ammoniaque (95-5-0,5). Le produit obtenu est agité dans l'éther ; essoré, séché à 50 °C sous 66 Pa. On obtient 3,9 g de produit souhaité, PF(cap): 162-164 °C.

**Exemple 29 à 31**

En opérant comme décrit dans l'exemple 28, ont été préparés les composés objet des exemples suivants :

29) Le 1-[(2-diméthylaminoéthyl)aminocarbonyl]-5,6-diméthyl-9-[(2-éthoxycarbonyléthyl) aminocarbonyloxy]-6H-pyrido[4,3-b]carbazole, PF(cap) : 120 °C (décomposition).

30) Le 1-[(2-diméthylaminoéthyl)aminocarbonyl]-5,6-diméthyl-9-[(3-benzyloxycarbonyl propyl)aminocarbonyloxy]-6H-pyrido[4,3-b]carbazole, PF(cap) : 125-128 °C.

31) Le 1-[(2-diméthylaminoéthyl)aminocarbonyl]-5,6-diméthyl-9-[(2-benzyloxycarbonyl éthyl)aminocarbonyloxy]-6H-pyrido[4,3-b]carbazole, PF(cap) : 112-114 °C.

**Exemple 32**

Le 1-[(2-diméthylaminoéthyl)aminocarbonyl]-5,6-diméthyl-9-(5-carboxypentanoyloxy)-6H-pyrido[4,3-b]carbazole

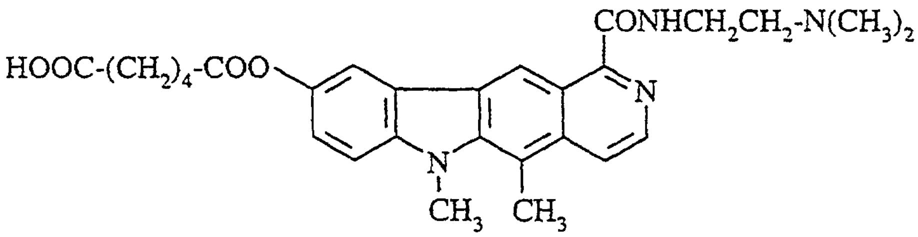

On hydrogène 2 g de 1-[(2-diméthylaminoéthyl)aminocarbonyl]-5,6-diméthyl-9-(5-benzyloxycarbonyl pentanoyloxy)-6H-pyrido[4,3-b]carbazole dissous dans 600 ml de méthanol en présence de 0,2 g de Palladium sur sulfate de baryum pendant 36 heures sous $3,5.10^5$ Pa d'hydrogène à température ambiante. On filtre le catalyseur et concentre à sec le filtrat. On reprend le résidu dans l'éthanol, concentre presque à sec et ajoute de l'éther éthylique. On essore le précipité et sèche à 40 °C sous 133 Pa. On obtient 1,9 g de produit souhaité, PF(cap) : 88-90 °C.

**Exemples 33-36**

En opérant comme décrit dans l'exemple 32 ont été préparés les composés objet des exemples suivants :

33) Le 1-[(2-diméthylaminoéthyl)aminocarbonyl]-5,6-diméthyl-9-[(2-carboxyéthylamino) carbonyloxy]-6H-pyrido [4,3-b]carbazole, PF(cap) 195-198 °C.

34) Le 1-[(2-diméthylaminoéthyl)aminocarbonyl]-5,6-diméthyl-9-[(3-carboxypropylamino) carbonyloxy]-6H-pyrido [4,3-b]carbazole, PF(cap) : 174-177 °C.

35) Le 1-[(2-diméthylaminoéthyl)aminocarbonyl]-5,6-diméthyl-9-(5-carboxy-2,2,5,5-tétraméthyl pentanoyloxy)-6H-pyrido[4,3-b]carbazole, PF (cap) : 191-193 °C.

36) Le 1-[(3-diméthylaminopropyl)aminocarbonyl]-5,6-diméthyl-9-[(4-carboxybutanoyloxy)-6H-pyrido[4,3-b]carbazole, P.F. (cap) : 222-228 °C.

**Exemple 37**

Le 1-[(2-diméthylaminoéthyl)aminocarbonyl]-5,6-diméthyl-9-(pipérazinocarbonyloxy)-6H-pyrido[4,3-b]carbazole

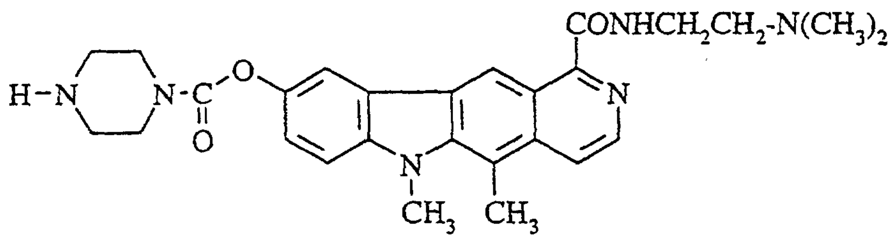

On porte au reflux 45 minutes 2,6 g de 1-[(2-diméthylaminoéthyl)aminocarbonyl]-5,6-diméthyl-9-[(4-benzyloxycarbonyl pipérazino)carbonyloxy]-6H-pyrido[4,3-b]carbazole, 5 ml de cyclohexène, 1 g de Palladium sur charbon à 10 % dans 260 ml de méthanol. On filtre le catalyseur et concentre à sec. On chromatographie le résidu sur silice en éluant avec un mélange de dichlorométhane, méthanol et ammoniaque (85-15-1). Le produit obtenu est lavé à

l'éther éthylique et à l'éthanol. On obtient 1,6 g de produit souhaité, PF(cap) : 230-234 °C.

**Exemple 38**

Le 1-[(2-diméthylaminoéthyl)arninocarbonyl]-5,6-diméthyl-9-(pyrid-3-yl carbonyloxy)-6H-pyrido[4,3-b]carbazole.

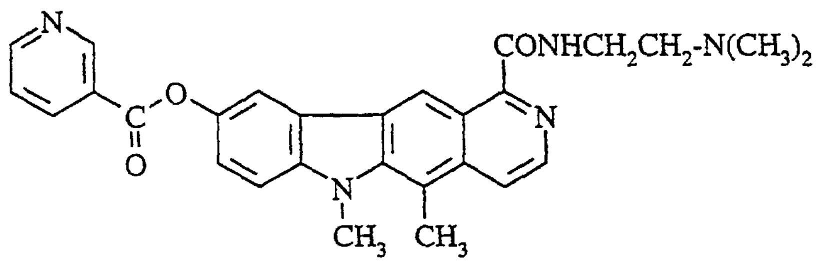

On agite pendant 16 heures à température ambiante un mélange contenant 3 g de 1-[(2-diméthylaminoéthyl) aminocarbonyl]-5,6-diméthyl-9-hydroxy-6H-pyrido[4,3-b]carbazole, 1,5 g d'acide nicotinique, 3,7 g de dicyclohexylcarbodiimide et 1,5 g de diméthylaminopyridine, dissous dans 100 ml de pyridine,. A l'issue des 16 heures, on ajoute à nouveau 2 g de dicyclohexylcarbodiimide et 0,75 g d'acide nicotinique et on agite 24 heures supplémentaires. On concentre à sec, reprend dans le dichlorométhane. On lave au bicarbonate de sodium, concentre la phase dichlorométhane de façon à éliminer le maximum de dicyclohexylurée formée par filtration et concentre à sec le filtrat. On chromatographie le résidu sur silice en éluant avec un mélange toluène, éthanol, triéthylamine (100,10,05). On obtient 3,5 g de produit souhaité, PF(cap) : 190-192 °C.

**Exemple 39:**ETUDE PHARMACOLOGIQUE

A/ Activité in vitro : cytotoxicité

Quatre lignées cellulaires ont été utilisées :

- 1 leucémie murine P388,
- 1 carcinome pulmonaire murin, le Lewis Lung Carcinoma (LLC),
- 1 carcinome épidermoïde humain, KB-3-1,
- la lignée résistante correspondante, KB-A1 dont la résistance multidrogue a été induite par l'adriamycine (ADR).

Les cellules sont cultivées dans du milieu de culture RPMI 1640 complet contenant 10 % de sérum de veau foetal, 2 mM de glutamine, 50 unités/ml de pénicilline, 50 µg/ml de streptomycine et 10 mM d'Hepes, pH = 7,4.

Les cellules sont réparties dans des microplaques et exposées aux composés cytotoxiques. Les cellules sont ensuite incubées pendant 2 jours (P388) ou 4 jours (LLC, KB-A1, KB-3-1). Le nombre de cellules viables est ensuite quantifié par un essai colorimétrique, le Microculture Tetrazolium Assay (Carmichael J., DeGraff W.G., Gazdar A.F., Minna J.D. and Mitchell J.R., Evaluation of a tetrazolium-based semiautomated colorimetric assay : assessment of chemosensitivity testing, Cancer Res., 47, 936-942, 1987).

Les résultats sont exprimés en IC50, concentration en cytotoxique qui inhibe à 50 % la prolifération des cellules traitées. Les résultats obtenus sur les lignées utilisées sont rassembles dans le tableau 1.

A titre d'exemple sont répertoriés ci-après les résultats obtenus avec les composés des exemples 2, 3 et 32 particulièrement représentatifs de l'invention et l'Adriamycine (ADR) pris comme produit de référence.

Tableau 1

| **Composés testés** | **IC50,(nM)** | | | |
|---|---|---|---|---|
| | **P388** | **LLC** | **KB-3-1** | **KB-A1** |
| **Exemple 2** | 5,8 | 13,5 | 36,1 | 758 |
| **Exemple 3** | 7,1 | 43,2 | 74,2 | 923 |

Tableau 1 (suite)

| Composés testés | IC50,(nM) | | | |
|---|---|---|---|---|
| | P388 | LLC | KB-3-1 | KB-A1 |
| Exemple 32 | 5,1 | 3,5 | 40,6 | 757,9 |
| ADR | 18,1 | 19,3 | 17,3 | 6693 |

La cytotoxicité des 3 composés testés est du même ordre de grandeur que celle de l'adriamycine sur les lignées sensibles. Sur la lignée KB-A1, résistante à l'adriamycine, les 3 composés testés sont 7 à 9 fois plus actifs que l'adriamycine. Ces dérivés peuvent donc être utilisés avec succès contre des tumeurs résistantes à l'adriamycine et présentant le phénotype de résistance multidrogue.

B/ Activité in vivo :

1/ *Activité antitumorale des composés sur la leucémie P388*

La lignée P388 (leucémie murine) a été fournie par le National Cancer Institute (Frederick, USA). Les cellules tumorales ($10^6$ cellules) ont été inoculées au jour 0 dans la cavité péritonéale de souris BDF1 femelles (Iffa-Credo, France) pesant de 18 à 20 g (groupes de 6 ou 7 animaux).

Les produits ont été administrés au jour 1 ou aux jours 1, 5, 9 aux doses indiquées, par voie intraveineuse.

L'activité antitumorale est exprimée en % de T/C :

$$\% \text{ T/C} = \frac{\text{Temps de survie médian des animaux traités}}{\text{Temps de survie médian des animaux contrôles}} \times 100$$

Les animaux survivants à 60 jours (survivants à long terme) sont indiqués.

A titre indicatif, sont répertoriés dans le tableau 2 les résultats obtenus avec le composé de l'exemple 32, particulièrement représentatif de l'invention.

Tableau 2

| Activité antitumorale du produit de l'exemple 32 administré par voie i.v. sur la leucémie P388 i.p. | |
|---|---|
| **Adminstration à J1 du composé de l'exemple 32 (mg/kg)** | **T/C % (survivants à J60)** |
| 0 | 100 |
| 20 | 125 |
| 40 | 154 |
| 80 | 186 |
| 160 | 233 |
| 240 | > 600 (4/7) |
| 320 | > 600 (6/7) |
| **Adminstration à J1, 5, 9 du composé de l'exemple 32 (mg/kg)** | **T/C % (survivants à J60)** |
| 0 | 100 |
| 10 | 137 |
| 20 | 171 |
| 40 | 236 |
| 80 | > 600 (6/7) |
| 160 | > 600 (6/7) |

L'examen de ce tableau montre que :

- Administré par voie i.v. à J1, le produit de l'exemple 32 est actif de 20 à 320 mg/kg et guérit 4 souris sur 7 à 240 mg/kg et 6 souris sur 7 à 320 mg/kg.

- Administré par voie i.v. aux jours 1, 5, 9 le produit de l'exemple 32 est actif de 10 à 160 mg/kg et guérit 6 souris sur 7 à 80 et 160 mg/kg.

- Le produit de l'exemple 32, même administré à très forte dose est bien toléré et n'induit ni perte de poids importante ni mortalité précoce. Il présente donc :

  . une toxicité générale très faible et
  . un excellent index thérapeutique.

2/ *Activité antitumorale des composés dans un modèle orthotopique de carcinome pulmonaire humain NCI-H460 greffé en intrapleural*

La lignée NCI-H460, qui provient d'un carcinome pulmonaire humain de type "non à petites cellules", a été fournie par l'American Type Culture Collection (Frederick, USA). Les cellules sont cultivées in vitro dans du milieu RPMI 1640 complet dans des flacons Falcon. Le jour de la greffe, les cellules sont détachées à l'aide de trypsine, centrifugées et mises en suspension dans du milieu de culture complet. Les cellules sont ensuite greffées dans la cavité pleurale de souris Nude Balb/C femelles ($10^6$ cellules par souris). Les produits sont administrés aux jours 7 et 14 par voie i.v., aux doses indiquées, à des groupes de 6 animaux. L'activité antitumorale est exprimée en % de T/C comme défini ci-dessus.

Sont répertoriés dans le tableau 3 les résultats obtenus avec le composé de l'exemple 3 de la présente demande (particulièrement représentatif de l'invention) et le composé le plus proche de l'état antérieur de la technique pris comme produit de référence.

Tableau 3

| **Activité antitumorale du composé de l'exemple 3 et du produit de référence administrés par voie i.v.sur le carcinome pulmonaire humain NCI-H460 greffé en intrapleural.** | | |
|---|---|---|
| **Composés testés** | **Doses mg/kg** | **T/C %** |
| Exemple 3 | 0 | 100 |
| | 56.5 | 133 |
| | 80 | 147 |
| | 113 | 174 |
| | . 160 | 181 |
| Produit de référence ** | 0 | 100 |
| | 40 | 126 |
| | 56.5 | 145 |
| | 80 | 156 |
| | 113* | 186 |

* dose toxique (1 souris morte pendant les traitements)

** le produit de référence est le dichlorhydrate de 1-[(2-diméthylaminoéthyl)aminocarbonyl]-5,6-diméthyl-9-hydroxy-6H-pyrido[4,3-b]carbazole, c'est à dire le composé, sous forme de dichlorhydrate, de l'exemple 1 de la demande de brevet EP 0 591 058 A1.

L'examen de ce tableau montre que le produit de référence est actif de 40 à 80 mg/kg, la dose de 113 mg/kg étant toxique. Le composé de l'exemple 3 est actif et très bien toléré, même à forte dose : administré 2 fois à 160 mg/kg, il augmente de 81 % la survie des animaux. L'index thérapeutique du composé de l'exemple 3 est donc excellent.

**Revendications**

1. Les composés de formule I:

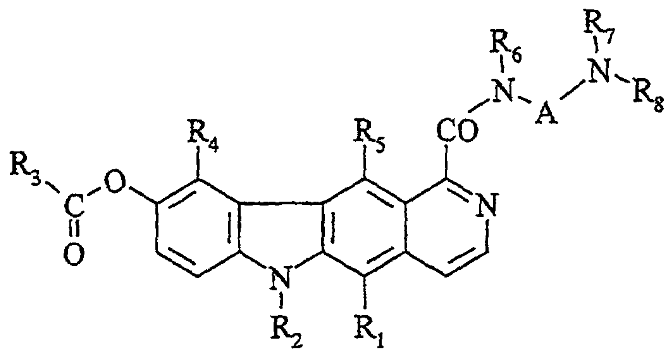

(I)

dans laquelle :

**$R_1$** représente un radical alkyle de 1 à 6 atomes de carbone en chaîne droite ou ramifiée ;

**$R_2$, $R_5$ et $R_6$,** identiques ou différents représentent chacun un atome d'hydrogène ou un radical alkyle, de 1 à 6 atomes de carbone, en chaîne droite ou ramifiée;

**$R_3$** représente :

A) un atome d'hydrogène,

B) un groupe ($C_3$-$C_8$)cycloalkyle, un groupe ($C_1$-$C_{20}$)alkyle, ($C_2$-$C_{20}$)-alkényle ou ($C_4$-$C_{20}$) -alkadiényle, chacun d'eux en chaîne droite ou ramifiée, et chacun de ces 4 groupes étant non substitué ou substitué par 1 à 3 groupes choisis parmi les radicaux :

a) ($C_1$-$C_6$)alkoxy ;
b) carboxy, ($C_1$-$C_6$)alkoxy-carbonyle, et benzyloxycarbonyle ;
c) aryle mono- bi- ou tri-cyclique, ou hétéroaryle, ce dernier est composé de 1 à 3 cycles penta et hexagonaux et comporte de 1 à 3 hétéroatomes choisis parmi les atomes d'azote, d'oxygène et de soufre;

C) un groupe aryle mono- bi- ou tri- cyclique éventuellement totalement ou partiellement hydrogéné, ou un groupe hétéroaryle, représentant un groupement pyridinyle ;
chacun des groupes aryle et hétéroaryle ci-dessus définis pouvant être éventuellement substitué par 1 à 3 substituants choisis parmi les radicaux :

a) ($C_1$-$C_6$)alkoxycarbonyle,
b) benzyloxycarbonyle,
c) ($C_1$-$C_6$)alkyle en chaine linéaire et ramifiée, non substitué et substitué par un et deux groupes phényles,
d) ($C_1$-$C_6$)alkoxy en chaine linéaire et ramifiée, et
e) carboxy ;

D) un groupe amino- mono- ou di-substitué par un ou deux substituants choisis parmi les radicaux :

a) ($C_1$-$C_6$)alkyles non substitués et substitués par un et deux substituants choisis chacun parmi les radicaux : carboxy, ($C_1$-$C_6$) alkoxycarbonyle et benzyloxycarbonyle,
b) aryle et hétéroaryle chacun mono- et bicyclique, ainsi que chacun d'eux partiellement et totalement hydrogénés tous étant non substitués et substitués par un et deux groupes choisis chacun parmi les radicaux carboxy, ($C_1$-$C_6$)alkoxycarbonyle et benzyloxycarbonyle ;

E) un groupe amino inclus dans un cycle penta ou hexagonal, renfermant éventuellement un second hétéroatome choisi parmi les atomes d'azote, oxygène et soufre, et éventuellement substitué par un ou deux groupes choisis chacun parmi les radicaux ($C_1$-$C_6$)alkyle, benzhydryle, ($C_1$-$C_6$)alkoxy, ($C_1$-$C_6$)alkoxycarbonyle, benzyloxycarbonyle, carboxy et

pipéridino ;

**$R_4$** représente :

- un atome d'hydrogène,
- un radical ($C_1$-$C_6$) alkyle, en chaîne droite ou ramifiée éventuellement substitué par un groupe di-($C_1$-$C_6$)alkylamino, ou
- un radical ($C_2$-$C_6$)-alkène ;

**$R_7$ et $R_8$**, identiques ou différents représentent chacun un atome d'hydrogène, un radical ($C_1$-$C_6$)alkyle ou ($C_2$-$C_6$)-alkényle, chacun en chaîne droite ou ramifiée, et $R_7$ et $R_8$ peuvent ensemble avec l'atome d'azote auquel ils sont liés former un hétérocycle, renfermant éventuellement un second hétéroatome choisi parmi les atomes d'azote, oxygène et soufre, et de plus $R_7$ peut être lié à $R_6$ afin de former ensemble un pont $-(CH_2)_m$-dans lequel m prend les valeurs 2 ou 3 ; et

A représente une chaine hydrocarbonée saturée linéaire ou ramifiée contenant de 1 à 10 atomes de carbone ;

ainsi que leurs isomères optiques, N-oxydes et sels d'addition, à un acide ou à une base, pharmaceutiquement acceptables.

**2.** Les composés de la revendication 1 répondant à la formule I dans laquelle:

- $R_1$ et $R_2$ représentent chacun un radical méthyle ;
- $R_3$ représente un groupe carboxyalkyle dans lequel la partie alkyle renferme de 1 à 8 atomes de carbone en chaîne droite ou ramifiée ;
- $R_4$ et $R_5$, identiques ou différents, représentent chacun un atome d'hydrogène ou un radical méthyle ;
- $R_6$ représente un atome d'hydrogène ;
- $R_7$ et Rg représentent chacun un radical méthyle et
- A représente $-(CH_2)_2$- ou $-(CH_2)_3$-;

ainsi que leurs isomères optiques, N-oxydes et sels d'addition, à un acide ou une base, pharmaceutiquement acceptables.

**3.** Un composé des revendications 1 et 2 qui est le 1-[(2-diméthylaminoéthyl)aminocarbonyl]-5,6-diméthyl-9-acétoxy-6H-pyrido[4,3-b] carbazole.

**4.** Un composé des revendications 1 et 2 qui est le dichlorhydrate de 1-[(2-diméthylaminoéthyl)aminocarbonyl]-5,6-diméthyl-9-(4-carboxybutanoyloxy)-6H-pyrido[4,3-b]carbazole.

**5.** Un composé des revendications 1 et 2 qui est le dichlorhydrate de 1-[(2-diméthylaminoéthyl)aminocarbonyl]-5,6,11-triméthyl-9-(5-carboxypentanoyloxy)-6H-pyrido [4,3-b]carbazole.

**6.** Un composé des revendications I et 2 qui est le 1-[(2-diméthylaminoéthyl)aminocarbonyl]-5,6-diméthyl-9-(5-carboxypentanoyloxy)-6H-pyrido[4,3-b]carbazole.

**7.** Le procédé de préparation des composés de la revendication 1 caractérisé en ce que :

- ♦ on estérifie un composé de formule II :

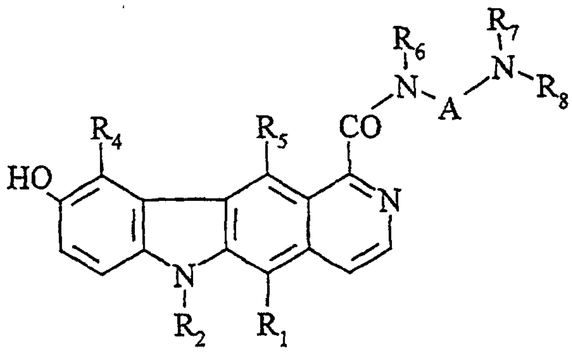

(II)

dans laquelle :

$R_1$, $R_2$, $R_4$, $R_5$, $R_6$, $R_7$, $R_8$ et A ont les significations définies dans la revendication 1,

au moyen d'un composé choisi parmi ceux correspondant aux formules :

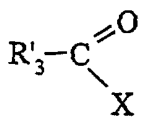

(IIIa)

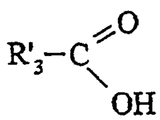

(IIIb)

et

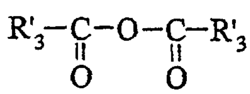

(IIIc)

dans lesquelles :

X représente un atome ou un groupe labile, et

$R'_3$ représente:

α) un groupe $(C_3-C_8)$cycloalkyle, un groupe $(C_1-C_{20})$alkyle, $(C_2-C_{20})$-alkényle ou $(C_4-C_{20})$ -alkadiényle, chacun d'eux en chaine droite ou ramifiée, et chacun de ces 4 groupes étant non substitué ou substitué par 1 à 3 groupes choisis parmi les radicaux :

a) $(C_1-C_6)$alkoxy;

b) $(C_1-C_6)$alkoxycarbonyle, et benzyloxycarbonyle ; et

c) aryle mono- bi- ou tri-cyclique, ou hétéroaryle, ce dernier est composé de 1 à 3 cycles penta et hexagonaux et comporte de 1 à 3 hétéroatomes choisis parmi les atomes d'azote, d'oxygène et de soufre;

β) un groupe aryle mono- bi- ou tri- cyclique éventuellement totalement ou partiellement hydrogéné, ou

un groupe hétéroaryle, représentant un groupement pyridinyle ;
chacun des groupes aryle et hétéroaryle ci-dessus définis pouvant être éventuellement substitué par 1 à 3 substituants choisis parmi les radicaux :

a) ($C_1$-$C_6$)alkoxycarbonyle,
b) benzyloxycarbonyle,
c) ($C_1$-$C_6$)alkyle en chaîne linéaire et ramifiée, non substitué et substitué par un et deux groupes phényles, et
d) ($C_1$-$C_6$)alkoxy en chaine linéaire et ramifiée ;

γ) un groupe amino- mono- ou di-substitué par un ou deux substituants choisis parmi les radicaux :

a) ($C_1$-$C_6$)alkyles non substitués et substitués par un et deux substituants choisis chacun parmi les radicaux : ($C_1$-$C_6$) alkoxycarbonyle et benzyloxycarbonyle, et
b) aryle et hétéroaryle chacun mono- et bicyclique, ainsi que chacun d'eux partiellement et totalement hydrogénés tous étant non substitués et substitués par un et deux groupes choisis chacun parmi les radicaux ($C_1$-$C_6$) alkoxycarbonyle et benzyloxycarbonyle ; et

δ) un groupe amino inclus dans un cycle penta ou hexagonal, renfermant éventuellement un second hétéroatome choisi parmi les atomes d'azote, oxygène et soufre, et éventuellement substitué par un ou deux groupes choisis chacun parmi les radicaux ($C_1$-$C_6$)alkyle, benzhydryle, ($C_1$-$C_6$)alkoxy, ($C_1$-$C_6$) alkoxycarbonyle, benzyloxycarbonyle et pipéridino ;

pour obtenir les composés de formule Ia :

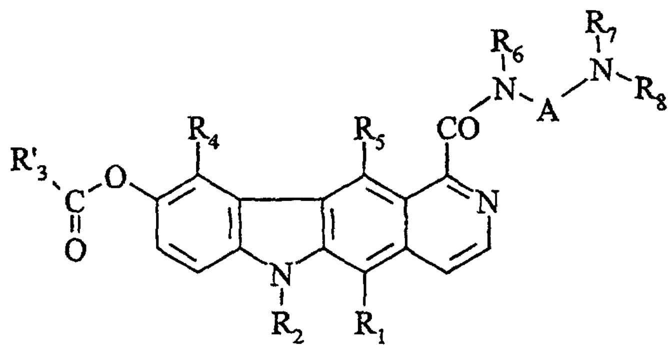

(Ia)

dans laquelle $R_1$, $R_2$, $R'_3$, $R_4$, $R_5$, $R_6$, $R_7$, $R_8$ et A ont les significations précédemment définies :

- on traite :
  les composés de formule Ia dans laquelle $R'_3$ renferme comme substituant au moins un groupe benzyloxy, c'est à dire les composés répondant plus précisément à la formule I'a :

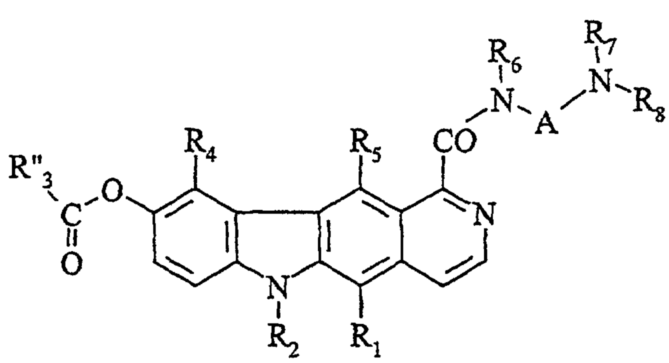

(I'a)

dans laquelle :

$R_1$, $R_2$, $R_4$, $R_5$, $R_6$, $R_7$, $R_8$ et A ont les significations précédemment définies et $R''_3$ représente :

α) un groupe $(C_3\text{-}C_8)$cycloalkyle, un groupe $(C_1\text{-}C_{20})$alkyle, en chaîne droite ou ramifiée, et chacun de ces groupes étant substitué par 1 à 3 radicaux benzyloxycarbonyles ;
β) un groupe aryle mono- bi- ou tri- cyclique éventuellement totalement ou partiellement hydrogéné, ou un groupe hétéroaryle, ce dernier est composé de 1 à 3 cycles penta et hexagonaux et comporte de 1 à 3 hétéroatomes choisis parmi les atomes d'azote, d'oxygène et de soufre;
chacun des groupes aryle et hétéroaryle ci-dessus définis étant substitué par 1 à 3 radicaux benzyloxycarbonyles ;
γ) un groupe amino- mono- ou di-substitué par un ou deux substituants choisis parmi les radicaux :

a) $(C_1\text{-}C_6)$alkyles substitués par un et deux radicaux benzyloxycarbonyles,
b) aryle et hétéroaryle chacun mono- et bicyclique, ainsi que chacun d'eux partiellement et totalement hydrogénés tous étant substitués par un et deux radicaux benzyloxycarbonyles ;

δ) un groupe amino inclus dans un cycle penta ou hexagonal, renfermant éventuellement un second hétéroatome choisi parmi les atomes d'azote, oxygène et soufre, et substitué par un ou deux radicaux benzyloxycarbonyles,

avec de l'hydrogène ou un donneur d'hydrogène pour obtenir les composés correspondants dans lesquels tout substituant benzyloxycarbonyle dans le composé de départ de formule I'a est remplacé par un radical carboxy, c'est à dire pour obtenir les composés de formule Ib :

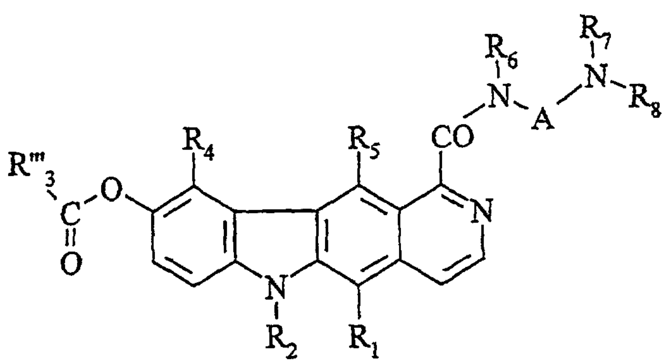

(Ib)

dans laquelle :

$R_1$, $R_2$, $R_4$, $R_5$, $R_6$, $R_7$, $R_8$ et A ont les significations précédemment définies et $R'''_3$ représente :

α) un groupe $(C_3\text{-}C_8)$cycloalkyle, un groupe $(C_1\text{-}C_{20})$alkyle en chaine droite ou ramifiée, et chacun de ces groupes étant substitué par 1 à 3 radicaux carboxy,
β) un groupe aryle mono- bi- ou tri- cyclique éventuellement totalement ou partiellement hydrogéné, ou un groupe hétéroaryle, ce dernier est composé de 1 à 3 cycles penta et hexagonaux et comporte de 1 à 3 hétéroatomes choisis parmi les atomes d'azote, d'oxygène et de soufre;
chacun des groupes aryle et hétéroaryle ci-dessus définis étant substitué par 1 à 3 radicaux carboxy ;
γ) un groupe amino- mono- ou di-substitué par un ou deux substituants choisis parmi les radicaux :

a) $(C_1\text{-}C_6)$alkyles substitués par un et deux radicaux carboxy,
b) aryle et hétéroaryle chacun mono- et bicyclique, ainsi que chacun d'eux partiellement et totalement hydrogénés tous étant substitués par un et deux radicaux carboxy ;

δ) un groupe amino inclus dans un cycle penta ou hexagonal, renfermant éventuellement un second hétéroatome choisi parmi les atomes d'azote, oxygène et soufre, et substitué par un ou deux radicaux carboxy ; et

- on fait réagir un composé de formule II telle que précédemment définie,

avec un mélange d'acide formique et d'anhydride acétique pour obtenir les composés de formule I dans laquelle $R_3$ représente un atome d'hydrogène c'est à dire pour obtenir les composés de formule Ic :

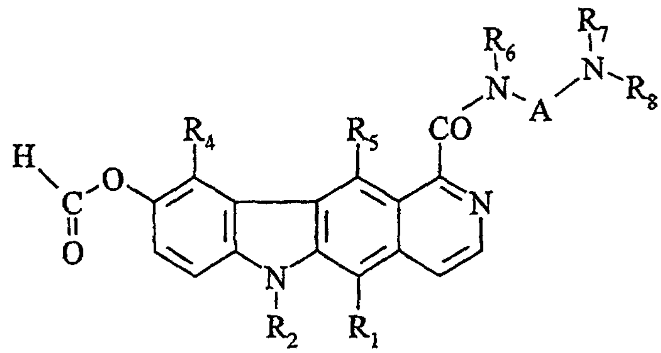

(Ic)

dans laquelle $R_1$, $R_2$, $R_4$, $R_5$, $R_6$, $R_7$, $R_8$ et A ont les significations précédemment définies.

**8.** Le procédé de préparation des composés de la revendication 1 répondant plus spécifiquement à la formule I':

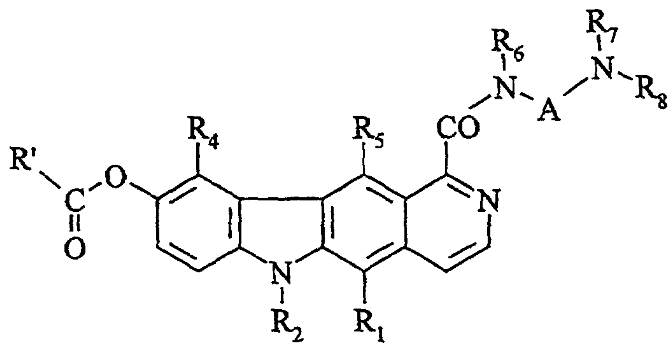

(I')

dans laquelle :

- $R_1$, $R_2$, $R_4$, $R_5$, $R_6$, $R_7$, $R_8$ et A ont les significations définies dans la revendication 1 et
- R' représente un radical $(C_1\text{-}C_{20})$alkyle substitué par un radical carboxy,

- caractérisé en ce que
- soit l'on traite les composés de formule I":

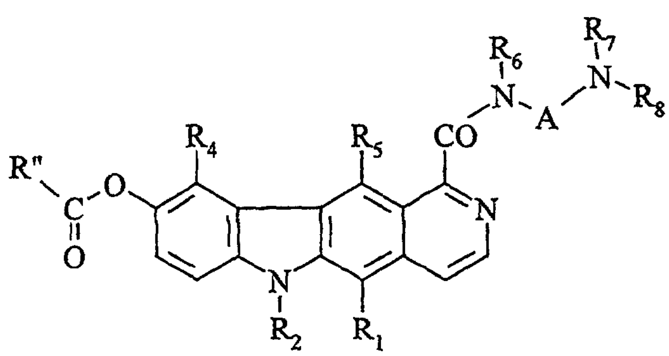

(I")

dans laquelle :

- $R_1$, $R_2$, $R_4$, $R_5$, $R_6$, $R_7$, $R_8$ et A ont les significations précédemment définies et

. R'' représente un radical ($C_1$-$C_{20}$)alkyle substitué par un radical benzyloxy,

avec de l'hydrogène ou un donneur d'hydrogène,

- soit on fait réagir un composé de formule II telle que définie dans la revendication 7 avec un anhydrique cyclique de formule :

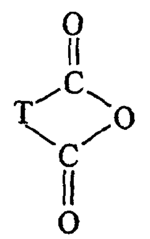

dans laquelle T représente une chaine hydrocarburée saturée de 2 à 10 atomes de carbone éventuellement substituée par 1 ou 2 radicaux alkyles de 1 à 5 atomes de carbone.

**9.** Le procédé de préparation des composés de la revendication 1 répondant plus spécifiquement à la formule I''':

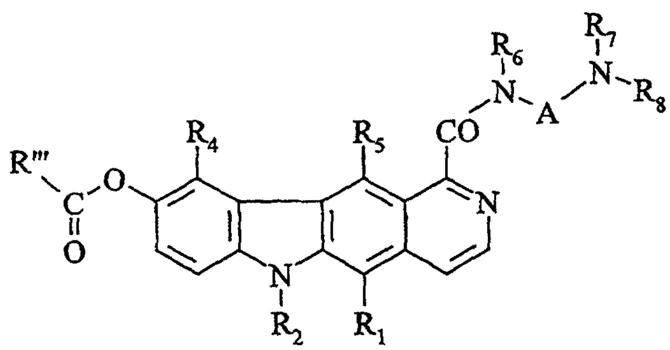

(I''')

dans laquelle :

. $R_1$, $R_2$, $R_4$, $R_5$, $R_6$, $R_7$, $R_8$ et A ont les significations définies dans la revendication 1 et
. R''' représente un groupe amino monosubstitué par :

soit un radical ($C_1$-$C_6$)alkyle non substitué ou substitué par un ou deux substituants choisis parmi les radicaux ($C_1$-$C_6$)alkoxycarbonyle et benzyloxycarbonyle,
soit un radical aryle ou hétéroaryle mono- ou bi-cyclique éventuellement totalement ou partiellement hydrogéné non substitué ou substitué par un ou deux groupes choisis chacun parmi les radicaux ($C_1$-$C_6$) alkoxycarbonyle et benzyloxycarbonyle,

caractérisé en ce que l'on fait réagir :

- un composé de formule II définie dans la revendication 7,
- avec un isocyanate de formule IV :

$$R_9\text{-}N{=}C{=}O \quad \text{(IV)}$$

dans laquelle :

. $R_9$ représente :

soit un radical ($C_1$-$C_6$)alkyle non substitué ou substitué par un ou deux substituants choisis parmi les radicaux ($C_1$-$C_6$)alkoxycarbonyle et benzyloxycarbonyle,
soit un radical aryle ou hétéroaryle mono- ou bi-cyclique éventuellement totalement ou partiellement hydrogéné, non substitué ou substitué par un ou deux groupes choisis chacun parmi les radicaux ($C_1$-$C_6$)alkoxycarbonyle et benzyloxycarbonyle.

**10.** Les compositions pharmaceutiques, utiles comme agents antitumoraux, contenant comme principe actif au moins un composé selon l'une quelconque des revendications 1 à 6, mélangé ou associé à un ou plusieurs excipients pharmaceutiques ou véhicules inertes non toxiques.

**Patentansprüche**

**1.** Verbindungen der Forme I:

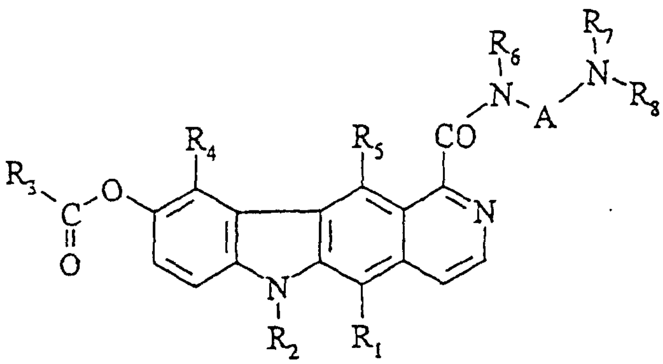

(I)

in der:

**$R_1$** eine Alkylgruppe mit 1 bis 6 Kohlenstoffatomen in gerader oder verzweigter Kette;
**$R_2$, $R_5$ und $R_6$,** die gleichartig oder verschieden sind, jeweils ein Wasserstoffatom oder eine Alkylgruppe mit 1 bis 6 Kohlenstoffatomen in gerader oder verzweigter Kette;
**$R_3$:**
A) ein Wasserstoffatom.
B) eine ($C_3$-$C_8$)-Cycloalkylgruppe, eine ($C_1$-$C_{20}$)-Alkylgruppe, eine ($C_2$-$C_{20}$)-Alkenylgruppe oder eine ($C_4$-$C_{20}$)-Alkadienylgruppe, jeweils mit gerader oder verzweigter Kette, wobei jede dieser Gruppen unsubstituiert oder mit 1 bis 3 Gruppen ausgewählt aus:

a) ($C_1$-$C_6$)-Alkoxygruppen;
b) Carboxygruppen, ($C_1$-$C_6$)-Alkoxy-carbonylgruppen und Benzyloxycarbonylgruppen; und
c) mono-, bi- oder tri-cyclische Arylgruppen oder Heteroarylgruppen, wobei die letzteren aus 1 bis 3 penta- und hexagonalen Ringen gebildet sind und 1 bis 3 Heteroatome ausgewählt aus Stickstoff-, Sauerstoff- und Schwefelatomen aufweisen;

substituiert ist;
C) eine mono-, bi- oder tri-cyclische, gegebenenfalls vollständig oder teilweise hydrierte Arylgruppe oder eine Heteroarylgruppe, die eine Pyridinylgruppe darstellt;
wobei jede der oben definierten Aryl- und Heteroarylgruppen gegebenenfalls durch 1 bis 3 Substituenten ausgewählt aus:

a) ($C_1$-$C_6$)-Alkoxycarbonylgruppen,
b) Benzyloxycarbonylgruppen,
c) ($C_1$-$C_6$)-Alkylgruppen mit gerader oder verzweigter Kette, die unsubstituiert oder durch eine oder zwei Phenylgruppen substituiert sind,
d) ($C_1$-$C_6$)-Alkoxygruppen mit gerader oder verzweigter Kette und
e) Carboxygruppen

substituiert ist, sein kann;
D) eine Aminogruppe, die mono- oder disubstituiert ist durch einen oder zwei Substituenten ausgewählt aus:

a) ($C_1$-$C_6$)-Alkylgruppen, die nicht substituiert oder durch einen oder zwei Substituenten ausgewählt aus: Carboxygruppen, ($C_1$-$C_6$)-Alkoxycarbonylgruppen und Benzyloxycarbonylgruppen substituiert sind,
b) Aryl- und Heteroarylgruppen, die jeweils mono- oder bicyclisch sind und die jeweils teilweise oder vollständig hydriert sind und unsubstituiert oder durch eine oder zwei Gruppen jeweils ausgewählt aus Carboxygruppen, ($C_1$-$C_6$)-Alkoxycarbonylgruppen und Benzyloxycarbonylgruppen substituiert sind;

E) eine Aminogruppe, die in einem penta- oder hexagonalen Ring enthalten ist, der gegebenenfalls ein zweites Heteroatom ausgewählt aus Stickstoff-, Sauerstoff- und Schwefelatomen enthält und gegebenenfalls durch eine oder zwei Gruppen jeweils ausgewählt aus ($C_1$-$C_6$)-Alkylgruppen, Benzhydrylgruppen, ($C_1$-$C_6$)-Alkoxygruppen, ($C_1$-$C_6$)-Alkoxycarbonylgruppen, Benzyloxycarbonylgruppen, Carboxygruppen und Piperidinogruppen substituiert ist;

**$R_4$:**
- ein Wasserstoffatom,
- eine ($C_1$-$C_6$)-Alkylgruppe mit gerader oder verzweigter Kette, die gegebenenfalls durch eine Di-($C_1$-$C_6$)-alkylaminogruppe substituiert ist, oder
- eine ($C_2$-$C_6$)-Alkengruppe;

**$R_7$ und $R_8$,** die gleichartig oder verschieden sind, jeweils ein Wasserstoffatom, eine ($C_1$-$C_6$)-Alkylgruppe oder eine ($C_2$-$C_6$)-Alkenylgruppe, jeweils mit gerader oder verzweigter Kette und $R_7$ und $R_8$ gemeinsam mit dem Stickstoffatom, an das sie gebunden sind, einen Heterocyclus bilden können, der gegebenenfalls ein zweites Heteroatom ausgewählt aus Stickstoff-, Sauerstoff- und Schwefelatomen enthält, und weiterhin $R_7$ mit $R_6$ verbunden sein kann unter gemeinsamer Bildung einer Brücke -$(CH_2)_m$-, worin m die Werte 2 oder 3 aufweist; und

**A** eine gesättigte, geradkettige oder verzweigte Kohlenwasserstoffkette mit 1 bis 10 Kohlenstoffatomen bedeuten;

deren optische Isomere, N-Oxide und Additionssalze mit einer pharmazeutisch annehmbaren Säure oder Base.

**2.** Verbindungen nach Anspruch 1 der Formel I, in der:

- $R_1$ und $R_2$ jeweils eine Methylgruppe;
- $R_3$ eine Carboxyalkylgruppe, deren Alkylteil 1 bis 8 Kohlenstoffatome in gerader oder verzweigter Kette enthält;
- $R_4$ und $R_5$, die gleichartig oder verschieden sind, jeweils ein Wasserstoffatom oder eine Methylgruppe;
- $R_6$ ein Wasserstoffatom;
- $R_7$ und $R_8$ jeweils eine Methylgruppe und
- A -$(CH_2)_2$- oder -$(CH_2)_3$- bedeuten;

sowie deren optische Isomere, N-Oxide und Additionssalze mit einer pharmazeutisch annehmbaren Säure oder Base.

**3.** Verbindung nach den Ansprüchen 1 und 2, nämlich 1-[2-(Dimethylaminoethyl)-aminocarbonyl]-5,6-dimethyl-9-acetoxy-6H-pyrido[4,3-b]carbazol.

**4.** Verbindung nach den Ansprüchen 1 und 2, nämlich 1-[(2-Dimethylaminoethyl)-aminocarbonyl]-5,6-dimethyl-9-(4-carboxybutanoyloxy)-6H-pyrido[4,3-b]carbazol-Dihydrochlorid.

**5.** Verbindung nach den Ansprüchen 1 und 2, nämlich 1-[(2-Dimethylaminoethyl)-aminocarbonyl]-5,6,11-trimethyl-9-(5-carboxypentanoyloxy)-6H-pyrido(4,3-b]carbazol-Dihydrochlorid.

**6.** Verbindung nach den Ansprüchen 1 und 2, nämlich 1-[(2-Dimethylaminoethyl)-aminocarbonyl]-5,6-dimethyl-9-(5-carboxypentanoyloxy)-6H-pyrido[4,3-b]-carbazol.

**7.** Verfahren zur Herstellung der Verbindungen nach Anspruch 1, dadurch gekennzeichnet, daß man:

- eine Verbindung der Formel II:

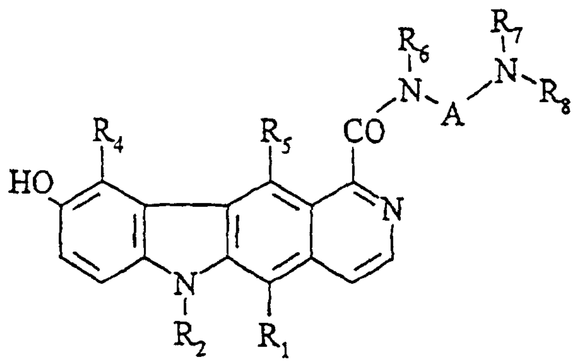

(II)

in der:
$R_1$, $R_2$, $R_4$, $R_5$, $R_6$, $R_7$, $R_8$ und A die in Anspruch 1 angegebenen Bedeutungen besitzen, mit Hilfe einer Verbindung der folgenden Formeln verestert:

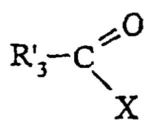

(IIIa)

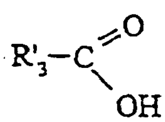

(IIIb)

und

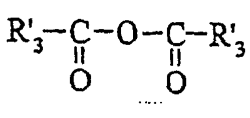

(IIIc)

in denen:

X ein labiles Atom oder eine labile Gruppe und

R'$_3$: α) eine ($C_3$-$C_8$)-Cycloalkylgruppe, eine ($C_1$-$C_{20}$)-Alkylgruppe, eine ($C_2$-$C_{20}$)-Alkenylgruppe oder eine ($C_4$-$C_{20}$)-Alkadienylgruppe, die jeweils in gerader oder verzweigter Kette vorliegen und wobei jede dieser Gruppen unsubstituiert oder substituiert ist durch 1 bis 3 Gruppen ausgewählt aus:

a) ($C_1$-$C_6$)-Alkoxygruppen;
b) ($C_1$-$C_6$)-Alkoxcarbonylgruppen und Benzyloxycarbonylgruppen; und

c) mono-, bi- oder tri-cyclische Arylgruppen oder Heteroarylgruppen, wobei die letzteren aus 1 bis 3 penta- und hexagonalen Ringen gebildet sind und 1 bis 3 Heteroatome ausgewählt aus Stickstoff-, Sauerstoff- und Schwefelatomen aufweisen;

β) eine mono-, bi- oder tri-cyclische Arylgruppe, die gegebenenfalls vollständig oder teilweise hydriert ist, oder eine Heteroarylgruppe, die eine Pyridinylgruppe darstellt;
wobei jede der oben definierten Aryl- und Heteroarylgruppen gegebenenfalls durch 1 bis 3 Substituenten ausgewählt aus:

a) ($C_1$-$C_6$)-Alkoxcarbonylgruppen,
b) Benzyloxycarbonylgruppen,
c) ($C_1$-$C_6$)-Alkylgruppen mit gerader oder verzweigter Kette, die unsubstituiert oder durch eine oder zwei Phenylgruppen substituiert sein können, und
d) ($C_1$-$C_6$)-Alkoxygruppen mit gerader oder verzweigter Kette substituiert sein kann;

γ) eine Aminogruppe, die mono- oder disubstituiert ist durch einen oder zwei Substituenten ausgewählt aus:

a) ($C_1$-$C_6$)-Alkylgruppen, die unsubstituiert sind oder durch einen oder zwei Substituenten jeweils ausgewählt aus: ($C_1$-$C_6$)-Alkoxcarbonylgruppen und Benzyloxycarbonylgruppen substituiert sind, und
b) Aryl- und Heteroarylgruppen, die jeweils mono- und bicyclisch sind, und die jeweils teilweise oder vollständig hydriert sein und unsubstituiert oder substituiert sein können durch eine oder zwei Gruppen jeweils ausgewählt aus ($C_1$-$C_6$)-Alkoxycarbonylgruppen und Benzyloxycarbonylgruppen; und

δ) eine Aminogruppe, die in einem penta- oder hexagonalen Ring enthalten ist, der gegebenenfalls ein zweites Heteroatom ausgewählt aus Stickstoff-, Sauerstoff- und Schwefelatomen enthält und gegebenenfalls durch eine oder zwei Gruppen jeweils ausgewählt aus ($C_1$-$C_6$)-Alkylgruppen, Benzhydrylgruppen, ($C_1$-$C_6$)-Alkoxygruppen, ($C_1$-$C_6$)-Alkoxycarbonylgruppen, Benzyloxycarbonylgruppen und Piperidinogruppen substituiert ist;

zur Bildung der Verbindungen der Formel Ia:

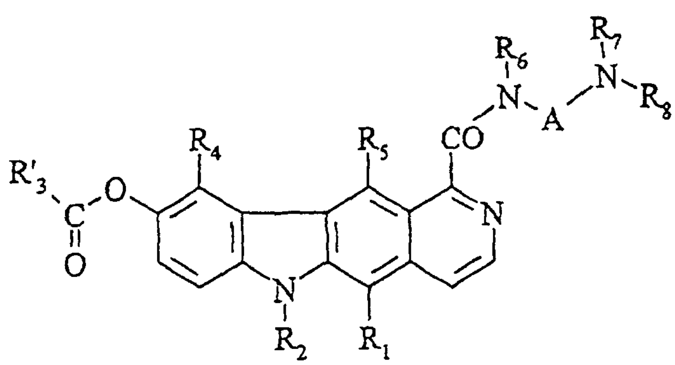

(Ia)

in der $R_1$, $R_2$, $R'_3$, $R_4$, R5, $R_6$, $R_7$, $R_8$ und A die oben angegebenen Bedeutungen besitzen:

- welche Verbindungen der Formel Ia, in der $R'_3$ als Substituenten mindestens eine Benzyloxygruppe darstellt, das heißt die Verbindungen, die genauer der Formel I'a entsprechen:

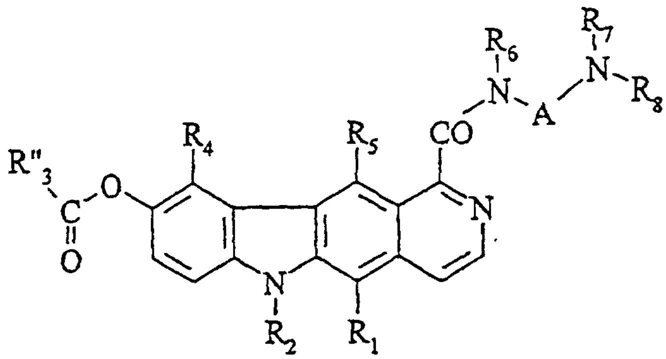

(I'a)

in der:
$R_1$, $R_2$, $R_4$, $R_5$, $R_6$, $R_7$, $R_8$ und A die oben angegebenen Bedeutungen besitzen und

$R''_3$: α) eine ($C_3$-$C_8$)-Cycloalkylgruppe, eine ($C_1$-$C_{20}$)-Alkylgruppe mit gerader oder verzweigter Kette, wobei jede dieser Gruppen durch 1 bis 3 Benzyloxycarbonylgruppen substituiert ist;
β) eine mono-, bi- oder tri-cyclische Arylgruppe, die gegebenenfalls vollständig oder teilweise hydriert ist oder eine Heteroarylgruppe, welchletztere aus 1 bis 3 penta- und hexagonalen Ringen gebildet ist und 1 bis 3 Heteroatome ausgewählt aus Stickstoff-, Sauerstoff- und Schwefelatomen aufweist,
wobei jede der oben definierten Aryl- und Heteroarylgruppen durch 1 bis 3 Benzyloxycarbonylgruppen substituiert ist;
γ) eine Aminogruppe, die mono- oder disubstituiert ist durch einen oder zwei Substituenten ausgewählt aus:

a) ($C_1$-$C_6$)-Alkylgruppen, die durch eine oder zwei Benzyloxycarbonylgruppen substituiert sind,
b) Aryl- und Heteroarylgruppen, die jeweils mono- oder bicyclisch sind und wobei jede dieser Gruppen teilweise oder vollständig hydriert ist und sämtlich durch eine oder zwei Benzyloxycarbonylgruppen substituiert sind;

δ) eine Aminogruppe, die in einem penta- oder hexagonalen Ring enthalten ist, der gegebenenfalls ein zweites Heteroatom ausgewählt aus Stickstoff-, Sauerstoff- und Schwefelatomen enthält, und durch eine oder zwei Benzyloxycarbonylgruppen substituiert ist,

man mit Wasserstoff oder einem Wasserstoffdonator behandelt zur Bildung der entsprechenden Verbindungen, worin sämtliche Benzyloxycarbonylsubstituenten der Ausgangsverbindung der Formel I'a durch eine Carboxygruppe ersetzt sind, das heißt zur Bildung der Verbindungen der Formel Ib:

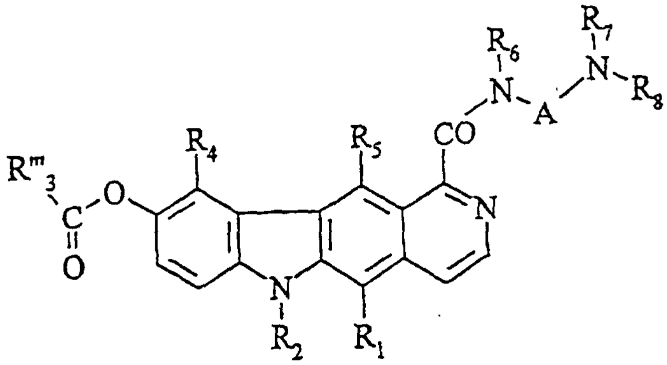

(Ib)

in der:

$R_1$, $R_2$, $R_4$, $R_5$, $R_6$, $R_7$, $R_8$ und A die oben angegebenen Bedeutungen besitzen und

R'''$_3$: α) eine ($C_3$-$C_8$)-Cycloalkylgruppe, eine ($C_1$-$C_{20}$)-Alkylgruppe mit gerader oder verzweigter Kette, wobei jede dieser Gruppen durch 1 bis 3 Carboxygruppen substituiert ist,
β) eine mono-, bi- oder tri-cyclische Arylgruppe, die gegebenenfalls vollständig oder teilweise hydriert ist, oder eine Heteroarylgruppe, welchletztere aus 1 bis 3 penta- oder hexagonalen Ringen gebildet ist und 1 bis 3 Heteroatome ausgewählt aus Stickstoff-, Sauerstoff- und Schwefelatomen enthält, wobei jede der oben definierten Aryl- und Heteroarylgruppen durch 1 bis 3 Carboxygruppen substituiert ist;
γ) eine Aminogruppe, die mono- oder disubstituiert ist durch einen oder zwei Substituenten ausgewählt aus:

a) ($C_1$-C6)-Alkylgruppen, die durch eine oder zwei Carboxygruppen substituiert sind;
b) Aryl- und Heteroarylgruppen, die mono- und bicyclisch sind und wobei jede dieser Gruppen teilweise oder vollständig hydriert ist und sämtlich durch eine oder zwei Carboxygruppen substituiert sind;

δ) eine Aminogruppe, die in einem penta- oder hexagonalen Ring enthalten ist, der gegebenenfalls ein zweites Heteroatom ausgewählt aus Stickstoff-, Sauerstoff- und Schwefelatomen enthält und durch eine oder zwei Carboxygruppen substituiert ist; und

- man eine Verbindung der oben definierten Formel II mit einer Mischung aus Ameisensäure und Essigsäureanhydrid behandelt zur Bildung der Verbindungen der Formel I, in der $R_3$ ein Wasserstoffatom darstellt, das heißt zur Bildung der Verbindungen der Formel Ic:

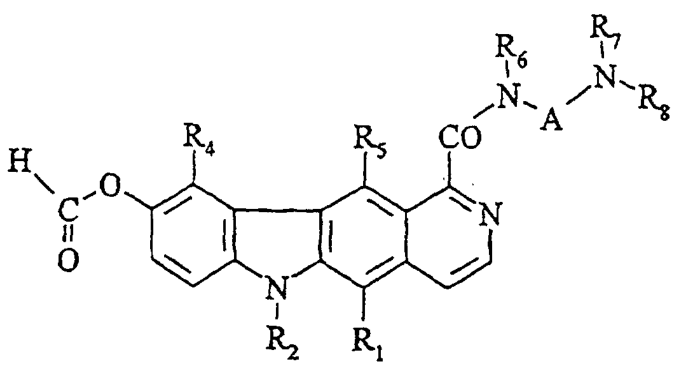

(Ic)

in der $R_1$, $R_2$, $R_4$, $R_5$, $R_6$, $R_7$, $R_8$ und A die oben angegebenen Bedeutungen besitzen.

**8.** Verfahren zur Herstellung der Verbindungen der Formel I, insbesondere der Formel I':

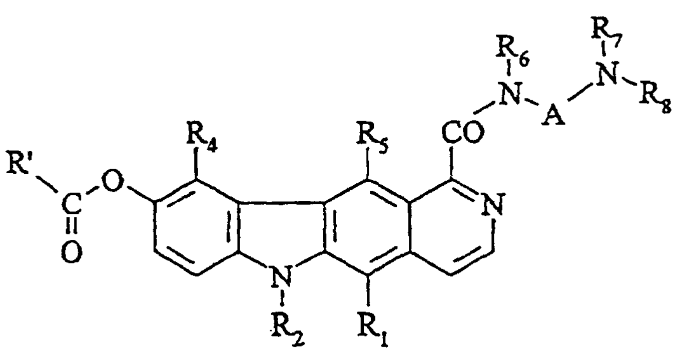

(I')

in der:

- $R_1$, $R_2$, $R_4$, $R_5$, $R_6$, $R_7$, $R_8$ und A die in Anspruch 1 angegebenen Bedeutungen besitzen und
- R' eine ($C_1$-$C_{20}$)-Alkylgruppe, die durch eine Carboxygruppe substituiert ist, bedeutet,

- dadurch gekennzeichnet, daß man
- entweder die Verbindungen der Formel I":

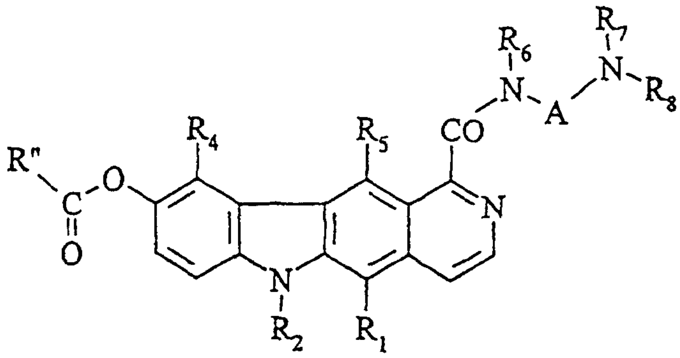

(I")

in der:

- $R_1$, $R_2$, $R_4$, $R_5$, $R_6$, $R_7$, $R_8$ und A die oben angegebenen Bedeutungen besitzen und
- R" eine ($C_1$-$C_{20}$)-Alkylgruppe, die durch eine Benzyloxygruppe substituiert ist, bedeutet,

mit Wasserstoff oder einem Wasserstoffdonator umsetzt,

- oder eine Verbindung der Formel II, wie sie in Anspruch 7 definiert worden ist, mit einem cyclischen Anhydrid der Formel:

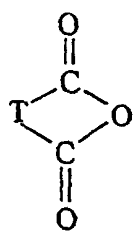

in der T eine gesättigte Kohlenwasserstoffkette mit 2 bis 10 Kohlenstoffatomen, die gegebenenfalls durch 1 oder 2 Alkylreste mit 1 bis 5 Kohlenstoffatomen substituiert ist, umsetzt.

**9.** Verfahren zur Herstellung der Verbindungen der Formel I, die insbesondere der Formel I"' entsprechen:

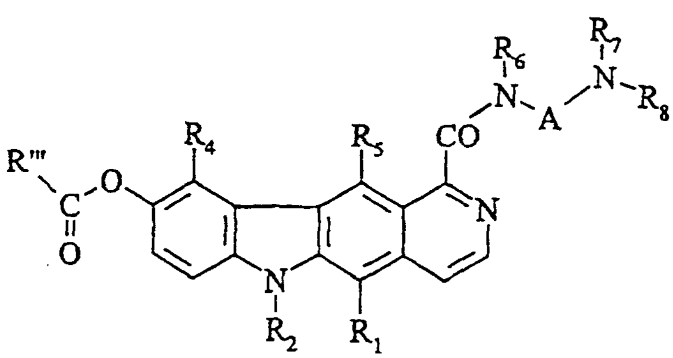

(I"')

in der:

- $R_1$, $R_2$, $R_4$, $R_5$, $R_6$, $R_7$, $R_8$ und A die in Anspruch 1 angegebenen Bedeutungen besitzen und
- R''' eine Aminogruppe bedeutet, die monosubstituiert ist durch:

entweder eine ($C_1$-$C_6$)-Alkylgruppe, die unsubstituiert ist oder durch einen oder zwei Substituenten ausgewählt aus ($C_1$-$C_6$)-Alkoxycarbonylgruppen und Benzyloxycarbonylgruppen substituiert ist, oder eine mono- oder bicyclische Aryl- oder Heteroarylgruppe, die gegebenenfalls vollständig oder teilweise hydriert ist und unsubstituiert ist oder substituiert ist durch eine oder zwei Gruppen jeweils ausgewählt aus ($C_1$-$C_6$)-Alkoxycarbonylgruppen und Benzyloxycarbonylgruppen bedeutet,

dadurch gekennzeichnet, daß man:

- eine Verbindung der Formel II, wie sie in Anspruch 7 definiert ist,
- mit einem Isocyanat der Formel IV umsetzt:

$$R_9 - N{=}C{=}O \qquad (IV)$$

in der:

- $R_9$:

entweder eine ($C_1$-$C_6$)-Alkylgruppe, die unsubstituiert oder durch einen oder zwei Substituenten ausgewählt aus ($C_1$-$C_6$)-Alkoxycarbonylgruppen und Benzyloxycarbonylgruppen substituiert ist, oder eine mono- oder bicyclische Aryl- oder Heteroarylgruppe, die gegebenenfalls vollständig oder teilweise hydriert ist und unsubstituiert oder substituiert ist durch eine oder zwei Gruppen jeweils ausgewählt aus ($C_1$-$C_6$)-Alkoxycarbonylgruppen und Benzyloxycarbonylgruppen, bedeutet.

**10.** Pharmazeutische Zubereitungen nützlich als antitumorale Mittel enthaltend als Wirkstoff mindestens eine Verbindung nach einem der Ansprüche 1 bis 6 in Mischung oder in Kombination mit einem oder mehreren inerten, nichttoxischen pharmazeutischen Trägermaterialien oder Bindemitteln.

## Claims

**1.** The compounds of formula I:

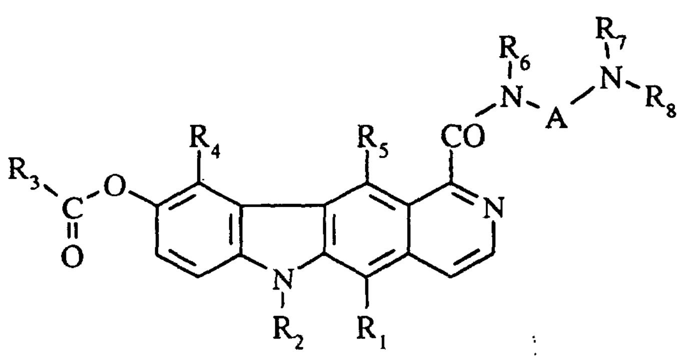

(I)

wherein:

**$R_1$** represents an alkyl radical having from 1 to 6 carbon atoms in a straight or branched chain;

**$R_2$, $R_5$ and $R_6$,** which may be the same or different, each represents a hydrogen atom or an alkyl radical having from 1 to 6 carbon atoms in a straight or branched chain;

**$R_3$** represents:

A) a hydrogen atom,

B) a $(C_3-C_8)$cycloalkyl group, or a $(C_1-C_{20})$alkyl, $(C_2-C_{20})$alkenyl or $(C_4-C_{20})$alkadienyl group, each of those being in a straight or branched chain, and each of the 4 groups being unsubstituted or substituted by from 1 to 3 groups selected from the radicals :

a) $(C_1-C_6)$alkoxy;

b) carboxy, $(C_1-C_6)$alkoxycarbonyl and benzyloxycarbonyl;

c) mono-, bi- or tri-cyclic aryl, or heteroaryl, the latter having from 1 to 3 rings each 5- or 6-membered and containing from 1 to 3 hetero atoms selected from the atoms nitrogen, oxygen and sulphur ;

C) a mono-, bi- or tri-cyclic aryl group that is optionally totally or partially hydrogenated, or a heteroaryl group, representing a pyridinyl group;

it being possible for each of the aryl and heteroaryl groups defined above optionally to be substituted by from 1 to 3 substituents selected from the radicals:

a) $(C_1-C_6)$alkoxycarbonyl,

b) benzyloxycarbonyl,

c) $(C_1-C_6)$alkyl each in a linear or branched chain, and each unsubstituted or substituted by one or two phenyl groups,

d) $(C_1-C_6)$alkoxy each in a linear or branched chain, and

e) carboxy;

D) an amino group that is mono- or di-substituted by one or two substituents selected from the radicals :

a) $(C_1-C_6)$alkyls that are each unsubstituted or substituted by one or two substituents each selected from the radicals : carboxy, $(C_1-C_6)$-alkoxycarbonyl and benzyloxycarbonyl,

b) aryl and heteroaryl, each being mono- or bi-cyclic, and each of those being partially or totally hydrogenated, and all being unsubstituted or substituted by one or two groups each selected from the radicals carboxy, $(C_1-C_6)$alkoxycarbonyl and benzyloxycarbonyl ;

E) an amino group that is included in a 5- or 6-membered ring, optionally containing a second hetero atom selected from the atoms nitrogen, oxygen and sulphur, and that is optionally substituted by one or two groups each selected from the radicals $(C_1-C_6)$alkyl, benzhydryl, . $(C_1-C_6)$alkoxy, $(C_1-C_6)$alkoxycarbonyl, benzyloxycarbonyl, carboxy and piperidino;

**$R_4$** represents:

- a hydrogen atom,
- a $(C_1-C_6)$alkyl radical in a straight or branched chain, optionally substituted by a di$(C_1-C_6)$alkylamino group, or
- a $(C_2-C_6)$alkene radical;

**$R_7$ and $R_8$,** which may be the same or different, each represents a hydrogen atom, or a $(C_1-C_6)$alkyl or $(C_2-C_6)$alkenyl radical each in a straight or branched chain, or

$R_7$ and $R_8$ may form, together with the nitrogen atom to which they are bonded, a heterocycle, optionally containing a second hetero atom selected from the atoms nitrogen, oxygen and sulphur, and, in addition, $R_7$ may be bonded to $R_6$ in order together to form a $-(CH_2)_m-$ bridge wherein m is 2 or 3; and

A represents a linear or branched saturated hydrocarbon chain having from 1 to 10 carbon atoms ;

and also their optical isomers, N-oxides, and pharmaceutically acceptable addition salts with an acid or a base.

**2.** Compounds of claim 1 corresponding to formula I wherein:

- each of $R_1$ and $R_2$ represents a methyl radical;
- $R_3$ represents a carboxyalkyl group in which the alkyl moiety has from 1 to 8 carbon atoms in a straight or branched chain;
- each of $R_4$ and $R_5$, which may be the same or different, represents a hydrogen atom or a methyl radical;
- $R_6$ represents a hydrogen atom;
- each of $R_7$ and $R_8$ represents a methyl radical, and
- A represents $-(CH_2)_2-$ or $-(CH_2)_3-$;

and also their optical isomers, N-oxides, and pharmaceutically acceptable addition salts with an acid or a base.

**3.** A compound of claims 1 and 2 which is 1-[(2-dimethylaminoethyl)aminocarbonyl]-5,6-dimethyl-9-acetoxy-6H-pyrido[4,3-b]carbazole.

**4.** A compound of claims 1 and 2 which is 1-[(2-dimethylaminoethyl)aminocarbonyl]-5,6-dimethyl-9-(4-carboxybutanoyloxy)-6H-pyrido[4,3-b]carbazole dihydrochloride.

**5.** A compound of claims 1 and 2 which is 1-[(2-dimethylaminoethyl)aminocarbonyl]-5,6,11-trimethyl-9-(5-carboxypentanoyloxy)-6H-pyrido[4,3-b]carbazole dihydrochloride.

**6.** A compound of claims 1 and 2 which is 1-[(2-dimethylaminoethyl)aminocarbonyl]-5,6-dimethyl-9-(5-carboxypentanoyloxy)-6H-pyrido[4,3-b]carbazole.

**7.** A process for the preparation of compounds of claim 1, characterised in that :

♦ a compound of formula II:

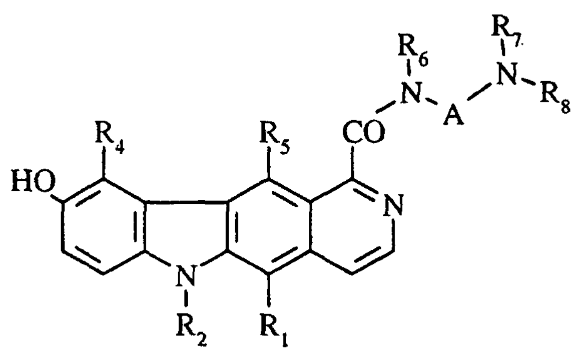

(II)

wherein:
$R_1$, $R_2$, $R_4$, $R_5$, $R_6$, $R_7$, $R_8$ and A are as defined in claim 1,
is esterified using a compound selected from those that correspond to the formulae :

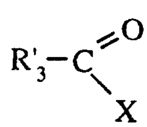

(IIIa)

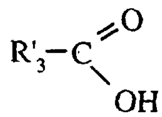 (IIIb)

and

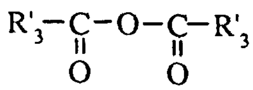 (IIIc)

wherein :

X represents a labile atom or group, and
R'$_3$ represents:

α) a ($C_3$-$C_8$)cycloalkyl group, or a ($C_1$-$C_{20}$)alkyl, ($C_2$-$C_{20}$)alkenyl or ($C_4$-$C_{20}$)alkadienyl group, each of those being in a straight or branched chain, and each of the 4 groups being unsubstituted or substituted by from 1 to 3 groups selected from the radicals :

a) ($C_1$-$C_6$)alkoxy;
b) ($C_1$-$C_6$)alkoxycarbonyl and benzyloxycarbonyl ; and
c) mono-, bi- or tri-cyclic aryl, or heteroaryl, the latter having from 1 to 3 rings each 5- or 6-membered and containing from 1 to 3 hetero atoms selected from the atoms nitrogen, oxygen and sulphur ;

β) a mono-, bi- or tri-cyclic aryl group that is optionally totally or partially hydrogenated, or a heteroaryl group, representing a pyridinyl group;
it being possible for each of the aryl and heteroaryl groups defined above optionally to be substituted by from 1 to 3 substituents selected from the radicals:

a) ($C_1$-$C_6$)alkoxycarbonyl,
b) benzyloxycarbonyl,
c) ($C_1$-$C_6$)alkyl each in a linear or branched chain, and each unsubstituted or substituted by one or two phenyl groups, and
d) ($C_1$-$C_6$)alkoxy each in a linear or branched chain ;

γ) an amino group that is mono- or di-substituted by one or two substituents selected from the radicals :

a) ($C_1$-$C_6$)alkyls that are each unsubstituted or substituted by one or two substituents each selected from the radicals: ($C_1$-$C_6$)-alkoxycarbonyl and benzyloxycarbonyl, and
b) aryl and heteroaryl, each being mono- or bi-cyclic, and each of those being partially or totally hydrogenated, and all being unsubstituted or substituted by one or two groups each selected from the radicals ($C_1$-$C_6$)-alkoxycarbonyl and benzyloxycarbonyl ; and

δ) an amino group that is included in a 5- or 6-membered ring, optionally containing a second hetero atom selected from the atoms nitrogen, oxygen and sulphur, and that is optionally substituted by one or two groups each selected from the radicals ($C_1$-$C_6$)alkyl, benzhydryl, ($C_1$-$C_6$)alkoxy, ($C_1$-$C_6$) alkoxy-carbonyl, benzyloxycarbonyl and piperidino ;

to obtain compounds of formula Ia :

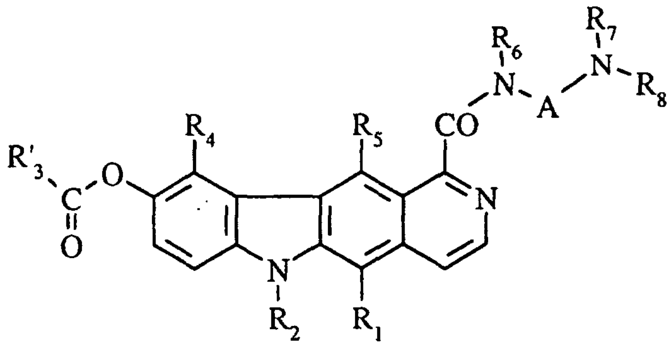

(Ia)

wherein $R_1$, $R_2$, $R'_3$, $R_4$, $R_5$, $R_6$, $R_7$, $R_8$ and A are as defined above :

- compounds of formula Ia wherein $R'_3$ includes as substituent at least one benzyloxy group, that is to say compounds that correspond more specifically to the formula I'a :

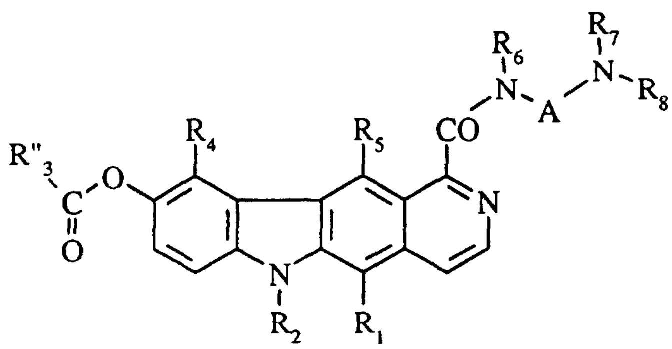

(I'a)

wherein :
$R_1$, $R_2$, $R_4$, $R_5$, $R_6$, $R_7$, $R_8$ and A are as defined above, and

$R''_3$ represents:

α) a ($C_3$-$C_8$)cycloalkyl group, or a ($C_1$-$C_{20}$)alkyl group in a straight or branched chain, each of those groups being substituted by from 1 to 3 benzyloxycarbonyl radicals ;
β) a mono-, bi- or tri-cyclic aryl group that is optionally totally or partially hydrogenated, or a heteroaryl group, the latter having from 1 to 3 rings each 5- or 6-membered and containing from 1 to 3 hetero atoms selected from the atoms nitrogen, oxygen and sulphur, each of the aryl and heteroaryl groups defined above being substituted by from 1 to 3 benzyloxycarbonyl radicals ;
γ) an amino group that is mono- or di-substituted by one or two substituents selected from the radicals :

a) ($C_1$-$C_6$)alkyls that are each substituted by one or two benzyloxycarbonyl radicals,
b) aryl and heteroaryl, each being mono- or bi-cyclic, and each of those being partially or totally hydrogenated, and all being substituted by one or two benzyloxycarbonyl radicals ;

δ) an amino group that is included in a 5- or 6-membered ring, optionally containing a second hetero atom selected from the atoms nitrogen, oxygen and sulphur, and that is substituted by one or two benzyloxycarbonyl radicals,

are treated with hydrogen or with a hydrogen donor to obtain the corresponding compounds wherein any benzyloxycarbonyl substituent in the starting compound of formula I'a has been replaced by a carboxy radical, that is to say to obtain compounds of formula Ib:

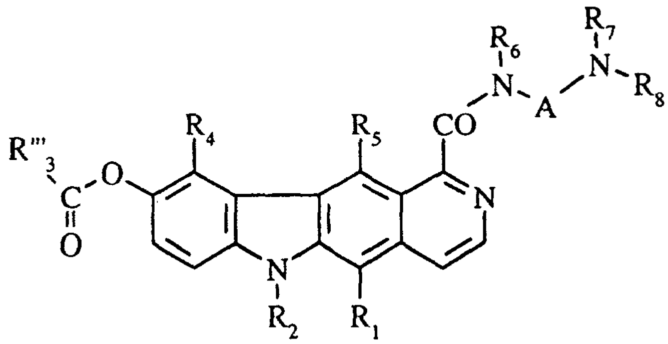

(Ib)

wherein :
$R_1$, $R_2$, $R_4$, $R_5$, $R_6$, $R_7$, $R_8$ and A are as defined above and

$R'''_3$ represents:

α) a ($C_3$-$C_8$)cycloalkyl group, or a ($C_1$-$C_{20}$)alkyl group in a straight or branched chain, each of those groups being substituted by from 1 to 3 carboxy radicals,
β) a mono-, bi- or tri-cyclic aryl group that is optionally totally or partially hydrogenated, or a heteroaryl group, the latter having from 1 to 3 rings each 5- or 6-membered and containing from 1 to 3 hetero atoms selected from the atoms nitrogen, oxygen and sulphur,
each of the aryl and heteroaryl groups defined above being substituted by from 1 to 3 carboxy radicals ;
γ) an amino group that is mono- or di-substituted by one or two substituents selected from the radicals:

a) ($C_1$-$C_6$)alkyls that are each substituted by one or two carboxy radicals,
b) aryl and heteroaryl, each being mono- or bi-cyclic, and each of those being partially or totally hydrogenated, and all being substituted by one or two carboxy radicals ;

δ) an amino group that is included in a 5- or 6-membered ring, optionally containing a second hetero atom selected from the atoms nitrogen, oxygen and sulphur, and that is substituted by one or two carboxy radicals;

and

♦ a compound of formula II as defined above is reacted
with a mixture of formic acid and acetic anhydride to obtain compounds of formula I wherein $R_3$ represents a hydrogen atom, that is to say to obtain compounds of formula Ic :

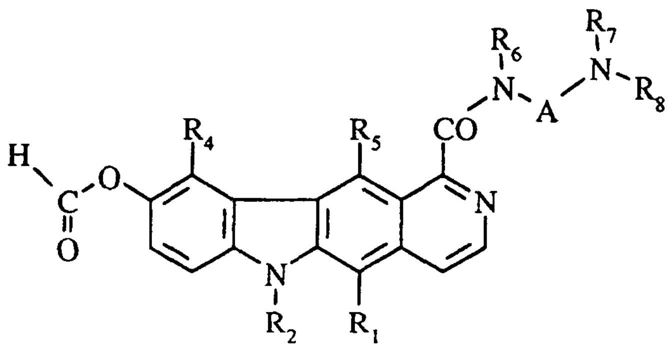

(Ic)

wherein $R_1$, $R_2$, $R_4$, $R_5$, $R_6$, $R_7$, $R_8$ and A are as defined above.

**8.** A process for the preparation of compounds of claim 1 that correspond more specifically to formula I':

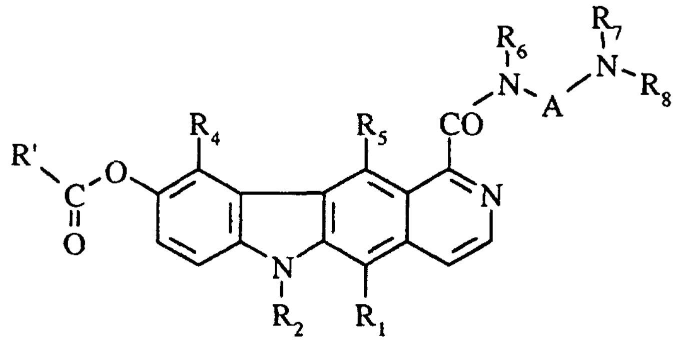

(I')

wherein :

- . $R_1$, $R_2$, $R_4$, $R_5$, $R_6$, $R_7$, $R_8$ and A are as defined in claim 1 and
- . R' represents a ($C_1$-$C_{20}$)alkyl radical that is substituted by a carboxy radical,

- characterised in that either
- compounds of formula I":

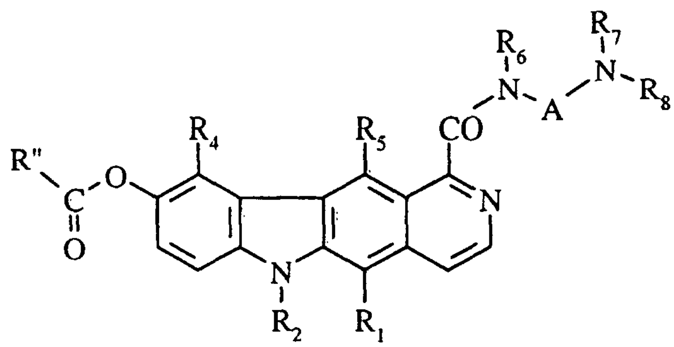

(I")

wherein :

- . $R_1$, $R_2$, $R_4$, $R_5$, $R_6$, $R_7$, $R_8$ and A are as defined above, and
- . R" represents a ($C_1$-$C_{20}$)alkyl radical that is substituted by a benzyloxy radical,

are treated with hydrogen or with a hydrogen donor, or

- a compound of formula II as defined in claim 7 is reacted with a cyclic anhydride of formula :

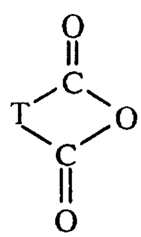

wherein T represents a saturated hydrocarbon chain having from 2 to 10 carbon atoms that is optionally substituted by 1 or 2 alkyl radicals having from 1 to 5 carbon atoms.

**9.** A process for the preparation of compounds of claim 1 that correspond more specifically to formula I'":

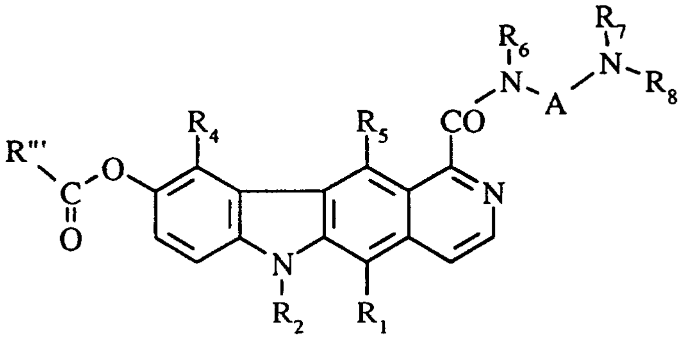

(I''')

wherein:

- . $R_1$, $R_2$, $R_4$, $R_5$, $R_6$, $R_7$, $R_8$ and A are as defined in claim 1, and
- . R''' represents an amino group that is monosubstituted by either:

a $(C_1\text{-}C_6)$alkyl radical that is unsubstituted or substituted by one or two substituents selected from the radicals $(C_1\text{-}C_6)$alkoxycarbonyl and benzyloxycarbonyl, or
a mono- or bi-cyclic aryl or heteroaryl radical that is optionally totally or partially hydrogenated and that is unsubstituted or substituted by one or two groups each selected from the radicals $(C_1\text{-}C_6)$alkoxycarbonyl and benzyloxycarbonyl,

characterised in that :

- a compound of formula II defined in claim 7 is reacted with
- an isocyanate of formula IV :

$$R_9\text{-}N{=}C{=}O \qquad (IV)$$

wherein :

- . $R_9$ represents either:

a $(C_1\text{-}C_6)$alkyl radical that is unsubstituted or substituted by one or two substituents selected from the radicals $(C_1\text{-}C_6)$alkoxycarbonyl and benzyloxycarbonyl, or
a mono- or bi-cyclic aryl or heteroaryl radical that is optionally totally or partially hydrogenated and that is unsubstituted or substituted by one or two groups each selected from the radicals $(C_1\text{-}C_6)$ alkoxycarbonyl and
benzyloxycarbonyl.

**10.** Pharmaceutical compositions, for use as anti-tumour agents, comprising as active ingredient at least one compound according to any one of claims 1 to 6, mixed with or in association with one or more pharmaceutical excipients or inert non-toxic carriers.